(19) Europäisches Patentamt
European Patent Office
Office européen des brevets

(11) EP 3 950 721 A1

(12) EUROPEAN PATENT APPLICATION

(43) Date of publication:
09.02.2022 Bulletin 2022/06

(21) Application number: 21192842.9

(22) Date of filing: 17.06.2011

(51) International Patent Classification (IPC):
C08B 37/00 (2006.01)   C12P 19/10 (2006.01)
C12R 1/645 (2006.01)   A61K 8/02 (2006.01)
A61K 8/73 (2006.01)   A61Q 19/00 (2006.01)
C08L 5/00 (2006.01)   C12N 1/14 (2006.01)

(52) Cooperative Patent Classification (CPC):
C12P 19/10; A61K 8/022; A61K 8/73; A61Q 19/00;
C08B 37/0018; C08L 5/00; C12N 1/145;
A61K 2800/10; C12R 2001/645

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 17.06.2010   JP 2010138793
06.07.2010   JP 2010154206

(62) Document number(s) of the earlier application(s) in
accordance with Art. 76 EPC:
11795838.9 / 2 583 982

(71) Applicant: Hayashibara Co., Ltd.
Okayama-shi, Okayama 702-8006 (JP)

(72) Inventors:
• SHIBUYA, Takashi
Okayama-shi, 7028006 (JP)
• IZAWA, Seisuke
Okayama-shi, 7028006 (JP)
• MIYAKE, Toshio
Okayama-shi, 7028006 (JP)

(74) Representative: Daniels, Jeffrey Nicholas
Page White & Farrer
Bedford House
John Street
London WC1N 2BF (GB)

Remarks:
•This application was filed on 24-08-21 as a divisional application to the application mentioned under INID code 62.
•Claims filed after the date of filing of the application (Rule 68(4) EPC).

(54) PULLULAN-CONTAINING POWDER

(57)   A particulate composition comprises pullulan and concomitant saccharides containing mannitol, and has the following features:

said pullulan and said concomitant saccharides are respectively insoluble and soluble in 75% by volume of methanol in water;

the content of said pullulan against the content of the total saccharides in the whole of said particulate composition is 97% by weight or higher, when determined based on the anthrone sulfuric acid method;

the content of said concomitant saccharides against the content of the total saccharides in the whole of said particulate composition is 3% by weight or lower, when determined based on the anthrone sulfuric acid method;

the content of said mannitol against the content of the total saccharides in the whole of said particulate composition does not exceed 2% by weight, on a dry solid basis; and

the weight-average molecular weight of said pullulan is 50,000 to 1,000,000 Daltons.

EP 3 950 721 A1

**Description**

Technical Field

The present invention relates to a particulate composition containing pullulan, process for producing the same, and uses thereof; and more particularly, to a novel particulate composition containing pullulan, which can be produced at a lesser cost without employing any complicated purification step and has advantageous features, process for producing the same, and uses thereof.

Background Art

As well known pullulan is a colorless and odorless water-soluble polysaccharide, a linear $\alpha$-glucan basically consisting of repeating units of maltotriose linked together in an $\alpha$-1, 6 linkage fashion, produced by a microorganism of the species *Aureobasidium pullulans*, a kind of fungus. Pullulan is a safe and edible polysaccharide produced from the microorganism usually by assimilating saccharides such as starch or starch syrup as carbon sources. Since pullulan has properties such as satisfactory water-solubility, relatively high adhesiveness, and improved film-formability, it has been extensively used in the fields of cosmetics and pharmaceuticals, as well as food products, in such a manner of using as an adhesive or coating material for food products and pharmaceuticals, a shaped products such as edible films and sheets dispersed with ingredients such as dyes, and as capsules encapsulated with granules containing functional ingredients used in the fields of food products and pharmaceuticals.

For example, Japanese Patent Publication No. 4291/84, and Japanese Patent Laid-OpenNos. 111817/84 and 1993-285969 disclose processes for producing pullulan films; Japanese Patent Laid-Open No. 155975/82 and Japanese Patent Tokuhyo No. 2009-539719 disclose uses of pullulan films for food packaging; Japanese Patent No. 3350823 discloses an adhesive composed of pullulan as a main ingredient; Japanese Patent Publication No. 6346/77 discloses a method for preventing oxidation using a pullulan film or pullulan coating; Japanese Patent Laid-Open No. 16217/85 discloses a film sweetener for diet prepared by dispersing a sweetener into a pullulan film; and Japanese Patent Publication No. 553/95 and Japanese Patent Laid-Open No. 2005-21124 disclose film preparations prepared by dispersing agents into pullulan; Satoshi NAKAMURA, "Yu-Kagaku" (Oil Chemicals), Vol. 34, No. 10, pp. 865-871, 1985, discloses an overview on the method for preparation of a pullulan membrane; and Satoshi NAKAMURA, "Japan Food Science", Vol. 25, No. 4, pp. 61-67, 1986, "Food Industry", Vol. 30, No. 10, pp. 33-39, 1987, Korin Publishing Co., Ltd., Tokyo, Japan, "Housou-Gijyutsu" (Packaging Technology), Vol. 29, No. 8, pp. 852-859, 1991, Yoshinao TANAKA, "Convertech", Vol. 28, No. 9, pp. 17-19, 2000, Kanou TAKEUCHI, and "Shoku-no-Kagaku" (Food Science), Vol. 277, March, pp. 96-99, 2001, Korin Publisher, Tokyo, Japan, introduce processes for producing pullulan films as edible films, and uses thereof.

In Japanese Patent Publication Nos. 36360/76, 42199/76, and 42635/80, the same applicant as the present invention has already disclosed processes for producing pullulan and particulate compositions containing pullulan on an industrial scale by culturing a microorganism of the species *Aureobasidium pullulans* in a culture medium containing, as carbon sources, saccharides such as starch syrup or partial starch hydrolyzate. Pullulan is formed in the culture of the above-identified microorganism, and the resulting culture containing pullulan is purified through the steps of removing the cells of the microorganims by centrifugation or filtration, and if necessary, after decoloring and desalting the resultant, adding thereunto an organic solvent such as methanol. Since pullulan is insoluble in methanol and concomitant saccharides other than pullulan are usually soluble in aqueous methanol, an organic solvent such as methanol is added to the above-identified culture to precipitate pullulan to separate into precipitant and supernatant. Thus, concomitant saccharides can be removed. By repeating these treatments of adding such organic solvent and separating the precipitant and the supernatant, pullulan is purified and improved its purity. The purified pullulan is prepared into a particulate composition containing pullulan via an appropriate pulverization step. Japanese Patent Laid-Open No. 21739/83 and Japanese Patent Nos. 27099/80 and 50665/83 disclose processes for producing shaped products such as films, coating membranes, sheets, and capsules which use the pullulan that has been purified and produced as in the above.

A relatively large amount of organic solvent is required in the purification step for pullulan by solvent precipitation where an organic solvent is added to a culture containing pullulan to precipitate pullulan which is then separated from the supernatant. Therefore, even if the used organic solvent is recovered by means of distillation, etc., the cost for apparatuses and organic solvents required for such recovering is not negligible, resulting in an unsatisfactory increment of its production cost. The purification step in itself for precipitating and separating pullulan is laborious and it requires time and effort, and after all this results in an increment of price of the resulting particulate composition containing pullulan. Because of these, the particulate composition containing pullulan produced by the above purification step is, for example, as disclosed by Satoshi NAKAMURA in "Yu-Kagaku" (Oil Chemicals), Vol. 34, No. 10, pp. 865-871, 1985, "Japan Food Science", Vol. 25, No. 4, pp. 61-67, 1986, "Food Industry", Vol. 30, No. 10, pp. 33-39, 1987, Korin Publishing Co., Ltd., Tokyo, Japan, and Japanese Patent Publication No. 49164/85, limited to specific uses for "reagent-grade pullulan" or "standard sample of pullulan" for molecular standard for assaying molecular weight, and "plasma adjuvant" in the field of pharmaceuticals. As molecular markers consisting of pullulan, Japanese Patent No. 3012917 discloses a pullulan separated and purified on high-performance liquid chromatography in normal-separation mode using a column packed with an amide-linked silica.

Considering the above circumstances, the same applicant as the present invention discloses in Japanese Patent No. 3232488 a process for producing a high pullulan content product with an increased pullulan content of up to at least about 50% by weight, on a dry solid basis (d.s.b.), preferably, about 55% to about 90% by weight. Since the product can be produced without employing any complicated purification step by solvent sedimentation, it has an advantageous feature that it can be produced easily at a lesser cost. In Japanese Patent No. 4203322, the same applicant as the present invention discloses a high pullulan content product with an improved stability against moisture change by adding thereunto α,α-trehalose; in Japanese Patent Laid-Open No. 2003-96103, the applicant discloses a particulate composition containing pullulan with an improved stability against moisture change by homogeneously adding thereunto a non-reducing saccharide consisting of glucoses as a constituent saccharide; and in Japanese Patent Laid-Open No. 2004-261132, the same applicant as the present invention discloses a process for producing pullulan with a reduced ratio of (weight-average molecular weight)/(number-average molecular weight).

Thereafter, the same applicant as the present invention developed the finding observed in the above-identified Japanese Patent No. 3232488 to explore a particulate composition containing pullulan with an increased pullulan content of up to at least 90% by weight without employing any purification step of solvent sedimentation, and commercialized two types of general-purpose particulate compositions containing pullulan called "PI-20" (commercialized by Hayashibara Shoji, Co., Ltd., Okayama, Japan) and "PF-20" (commercialized by Hayashibara Shoji, Co., Ltd., Okayama, Japan) directed for use in food products, pharmaceuticals, and cosmetics (see Satoshi NAKAMURA in "Yu-Kagaku" (Oil Chemicals), Vol. 34, No. 10, pp. 865-871, 1985, "Japan Food Science", Vol. 25, No. 4, pp. 61-67, 1986, and "Shokuhin-Kogyo" (Food Industry), Vol. 30, No. 10, pp. 33-39, 1987, Korin Publishing Co., Ltd., Tokyo, Japan). Thereafter, these two types of particulate compositions were integrated into "Food Additive Pullulan" (commercialized by Hayashibara Shoji, Co., Ltd., Okayama, Japan) being substantially equivalent to "PI-20", and the "Food Additive Pullulan" has now been commercialized. Since the "Food Additive Pullulan" has a satisfactory adhesive power, adherence, membrane-formability, and lubricity, as well as safeness and relatively low price, it has been extensively used as general-purpose particulate compositions containing pullulan; food viscosity-imparting agents, bonds, adhesives, and coating agents; instantly water-soluble solid-color films after processing; print films being printed with edible inks; and as blended films kneaded with food products, flavors, etc.

According to the subsequent findings of the present inventors, varying depending on their thickness, pullulan films prepared with the above-identified "Food Additive Pullulan" have various improved properties but have the following defects of causing occasional cracking or rupturing to become unusable when a stress concentrated site were formed by folding, inflecting or the like. Although the "Food Additive Pullulan" reaches its pullulan content of at least 90% by weight, the content or the molecular distribution of pullulan contained therein is not constant depending on its production lot, and thus it has the defect that products with constant properties of solubility and adhesive power could hardly be obtained. According to the confirmation by the inventors of the present invention, the pullulan content in "Food Additive Pullulan" is at least 90% by weight; however, it changes usually in the range of about 90% to about 96% by weight, depending on its production lot. Following this change, the content of concomitant saccharides other than pullulan will change in the range of about 4% to about 10% by weight. When the fluctuation range of the content of concomitant saccharides changes even by two times or higher, it naturally influences on the solubility and adhesiveness of a particulate composition containing pullulan, resulting in the defect that a desired product with constant solubility, adhesive power, or the like could hardly be obtained. Similarly, when the molecular distribution of pullulan contained in the particulate composition changes, it influences on the solubility and adhesive power of the pullulan *per se*, and this, along with the change of the content of concomitant saccharides, inevitably causes the change of solubility and adhesiveness of the particulate composition. Because of this, when "Food Additive Pullulan" is used in shaped products including films and the properties such as strength, solubility, and disintegrability of the shaped products obtained therewith are extreme cases, the shaped products are different each other depending on the production lot of "Food Additive Pullulan". For example, pharmaceuticals are required that the dynamics of their effective ingredients in living bodies such as absorption, distribution, metabolism, and excretion should be constant after their administrations. Also, pharmaceutical additives used for such pharmaceuticals should be those which do not affect pharmacological effects but stably maintain their dispositions. However, when conventional "Food Additive Pullulan" is used as a pharmaceutical additive, for example, as an adhesive agent for tablets, etc., as pharmaceuticals, the dissolution rate of the effective ingredients of the pharmaceuticals could not be constant depending on its production lot, and this has a fear of affecting pharmacokinetics in living bodies. Thus, putting for use as food products aside, "Food Additive Pullulan" should not necessarily be sufficient enough for an additive for use as pharmaceuticals which require persistent properties such as strength, solubility, and adhesive power and also require a constant disposition; or for use as quasi-drugs and cosmetics. The above-identified defect, inherent to "Food Additive Pullulan" as a general particulate composition containing pullulan, should essentially be overcome to more expand the use of pullulan or shaped product containing pullulan to the fields of food products, as well as pharmaceuticals, quasi-drugs, and cosmetics, which are required to be produced in such a manner of keeping constant or persistent quality without dispersion, independently of their production lots; or further to expand the use of pullulan or shaped products containing pullulan to the field of uses where a higher strength is required for such shaped products containing pullulan, for example, pullulan films even in the field of food products where "Food Additive Pullulan" has been conventionally used.

Disclosure of Invention

The present invention, which was made to solve the above defect residing in conventional particulate composition containing pullulan, aims to provide a particulate composition containing pullulan that is produced without employing any complicated purification step by solvent sedimentation or the like, has a higher rupture strength than those of conventional ones when formed into a film, has a constant composition retaining stably its concomitant saccharide content to an extremely low level, and preferably, has a molecular distribution of pullulan contained therein; and to provide uses thereof.

The present inventors energetically studied to solve the above object and repeatedly took a process of trial and error to find specific mutants of a microorganism of the species *Aureobasidium pullulans,* which yield pullulan in an amount substantially equal to that of the parent strain but have a significantly lesser ratio of saccharides other than pullulan, i.e., concomitant saccharides, among the artificially produced microorganisms of the species *Aureobasidium pullulans* when cultured in a selection medium containing glucose and maltose as carbon sources. Among these mutants, the one with the minimum ratio of concomitant saccharides was selected, cultured in a culture medium containing glucose and maltose as carbon sources, followed by producing a particulate composition containing pullulan in usual manner without employing any purification step of solvent precipitation for precipitating pullulan with an organic solvent. Thus, there is obtained a particulate composition containing pullulan with a significantly lesser amount of mannitol, a metabolite of a microorganism of the species *Aureobasidium pullulans*, compared to the above-identified "Food Additive Pullulan". When a pullulan film was formed with the particulate composition in usual manner, the rupture strength was significantly higher than that obtained with the above "Food Additive Pullulan". In addition, the concomitant saccharide content in the particulate composition remains stably at an extremely low level independently of its production lot, the saccharide composition remains roughly stable, and the molecular distribution of pullulan contained in the composition remains stably within a constant range. Therefore, the particulate composition can keep its quality at a constant level in terms of the above rupture strength, as well as solubility against water and adhesive power to bind other substances each other. Even when formed into shaped products such as pharmaceuticals, quasi-drugs, and cosmetics in the form of a film, sheet, tablet or granule, the particulate composition affords the shaped products a constant quality in terms of physical properties such as strength, solubility, and disintegrability. Thus, the particulate composition containing pullulan can form shaped products with constant and persistent physical properties required for pharmaceuticals, quasi-drugs, cosmetics, etc., and particularly, even when used in pharmaceuticals, the particulate composition can be used as a pharmaceutical additive because it has no fear of affecting disposition of effective ingredients that is inducible by dispersion of dissolution or disintegration. The present invention was made based on these findings.

The present invention solves the above object by providing a particulate composition containing pullulan characterized in that it is produced by culturing a mutant of a microorganism of the species *Aureobasidium pullulans* in a culture medium containing glucose and maltose as carbon sources without employing a step of removing concomitant saccharides from the resulting culture medium; it consists of a pullulan fraction insoluble in 75% by volume of methanol in water and a fraction of concomitant saccharides soluble in the aqueous methanol solution; it has a ratio of 3% by weight or lower of concomitant saccharides in terms of the concomitant saccharide content against the total content of sugars contained in the whole particulate composition, determined based on the anthrone-sulfuric acid method; and that it contains mannitol.

As described above, the particulate composition containing pullulan has a significant feature of that it has a lesser ratio of 3% by weight or lower of concomitant saccharides in terms of the concomitant saccharide content against the total content of sugars contained in the whole particulate composition, and the above total saccharide content and the concomitant saccharide content are both determined based on the anthrone-sulfuric acid method. As well known, the anthrone-sulfuric acid method is an assay system for saccharides where anthrone color reaction is utilized, and the absorbance of the color reaction is obtained as an amount proportional to the saccharide content, and therefore the saccharide content can be obtained based on the data. In the case of determining saccharides which consist of glucose, they can be determined in terms of D-glucose by using D-glucose as a standard substance. The anthrone-sulfuric acid method is employed as an assay for "Monosaccharides and Oligosaccharides" in purification test for pullulan as disclosed in Japanese Standard of Food Additives" (8th edition), published by Japan Food Additives Association (JAFA), pp. 572-573 (see the column of "Pullulan"), 2007. Since the method described in the above-identified "Japanese Standard of Food Additives" is a widely recognized standard method, in the present invention, as disclosed in the later described Experiment 2-3, the ratio of the content of concomitant saccharides against the content of total saccharides contained in the whole particulate composition is determined in accordance with the assay of "Monosaccharides and Oligosaccharides" described in the above "Japanese Standard of Food Additives".

Among saccharides, for example, like the later described mannitol, there exist saccharides that are not colored and undetectable by the anthrone-sulfuric acid method, and the total saccharide content and the concomitant saccharide content do not necessarily correspond exactly with the total saccharide content and the concomitant saccharide content. In the present specification, however, the total saccharide content determined based on the anthrone-sulfuric acid method is simply called "total saccharide content", unless specified otherwise; and also the concomitant saccharide content in the concomitant saccharide fraction, determined based on the method, is simply called "concomitant saccharide content".

The pullulan content in a particulate composition containing pullulan can be determined by assaying the pullulan content in a pullulan fraction based on the anthrone-sulfuric acid method in accordance with the assay for concomitant saccharide content, however, since the particulate composition containing pullulan of the present invention consists of a pullulan fraction and a concomitant saccharide fraction, the pullulan content of the particulate composition can be easily determined by subtracting the above concomitant saccharide content from the above total saccharide content. Accordingly, the ratio of the concomitant saccharide content against the total saccharide content is 3% by weight or lower, and this means that the pullulan content in a pullulan fraction is 97% by weight or higher. As a matter of course, the pullulan content does not contain saccharides such as mannitol that are not detected by the anthrone-sulfuric acid method. Accordingly, since the particulate composition containing pullulan of the present invention contains concomitant saccharides whose maximum level is extremely as low as 3% by weight of the total saccharide content, if the concomitant saccharide content varies from over 0% by weight to 3% by weight, the changing ratio against the remaining 97% by weight or higher of pullulan is relatively low, and therefore the total saccharide composition of the particulate composition containing pullulan of the present invention is roughly constant as a whole.

As described above, the particulate composition of the present invention has another maj or feature that it contains mannitol. Varying depending on the types of carbon sources used in culture, mannitol is a substance that is contained in the culture as a metabolite of a microorganism of the species *Aureobasidium pullulans.* However, mannitol is soluble in aqueous methanol and is removed when the resulting culture is purified by solvent precipitation using an organic solvent(s). Accordingly, the fact that mannitol is not removed from the particulate composition and contained therein means that the particulate composition containing pullulan of the present invention is of that produced without employing a step of removing concomitant saccharides by solvent precipitation using organic solvents, etc. The particulate composition usually contains about 2% by weight, d.s.b., of saccharides that are not colored and detected by the anthrone-sulfuric acid method, and the main ingredient is mannitol.

For comparison, mannitol is quantified, for example, on high-performance liquid chromatography (HPLC) analysis shown in the later described Experiment 4. Since the detection limit of mannitol on the HPLC analysis is usually speculated to be about 0.01% by weight, the fact that the particulate composition containing pullulan of the present invention means that it contains at least 0.01% by weight, d.s.b., of mannitol. Depending on the variety of the types of carbon sources used in culture, the content of mannitol in the particulate composition containing pullulan of the present invention does not usually exceed 2% by weight, d.s.b. Mannitol dissolves in 75% by volume of methanol in water and usually it is contained in a concomitant saccharide fraction, however, as mentioned above, mannitol is not contained in the content of concomitant saccharides because it is not detected on the anthrone-sulfuric acid method.

As described above, nevertheless, the particulate composition containing pullulan of the present invention contains mannitol and is produced without employing a step of removing concomitant saccharides by solvent precipitation using an organic solvent(s), etc., the concomitant saccharide content is extremely as low as 3% by weight or lower. Accordingly, the particulate composition, which contains mannitol and has an extremely lower amount of concomitant saccharides, has not yet been provided before the present invention was made as far as the applicant of the present invention knows and thus it is a novel particulate composition containing pullulan.

The particulate composition containing pullulan of the present invention can be formed into a film with a thickness of 100 μm, more particularly, a film with a thickness of 100 μm is obtained by casting a 20% w/v aqueous solution of the particulate composition on a plain plate, drying the casted solution at 25°C, and allowing the dried product to stand at a relative humidity of 22%. The film thus obtained exhibits a rupture strength of piercing of 20 MPa or higher on the piercing test for rupture strength using an adaptor for piercing test having a sectional area of 1 mm$^2$. As shown in the later described Experiment, the above-identified rupture strength of piercing is significantly higher than that obtained with the above-identified "Food Additive Pullulan". The fact shows that the particulate composition containing pullulan of the present invention has an advantageously significant feature that it has a relatively high rupture strength of piercing when formed into a film, though the particulate composition is so called "General-Purpose Industrial Product" produced without employing any purification step for pullulan, such as solvent sedimentation or the like.

The present invention solves the above object by providing a process for producing a particulate composition containing pullulan characterized in that it contains the steps of culturing a mutant of a microorganism of the species *Aureobasidium pullulans* in a culture medium containing glucose and maltose as carbon sources; removing the cells from the resulting culture; and decoloring, desalting, concentrating, and pulverizing the resultant, without employing a step of removing concomitant saccharides.

Anymutants of amicroorganismof the species *Aureobasidium pullulans* can be used in the process of the present invention as long as they can produce the particulate composition containing pullulan of the present invention without employing any removing step for removing concomitant saccharides, and they should not be limited to specific ones. Preferred examples of such mutants include the later described mutant of *Aureobasidium pullulans* S-2 strain (deposited in International Patent Organism Depositary National Institute of Advanced Industrial Science and Technology under the accession number of FERM BP-11261). Based on the disclosure of the present specification, these mutants can be obtained by using the above S-2 strain as a parent strain and applying thereto conventional mutation method and the later described screening method. As shown in the later described Experiment 1, S-2 strain is randomly mutated by conventional mutation method, followed by screening the resulting mutants with an index of the content of concomitant saccharides other than pullulan contained in the resulting cultures, and selecting the ones with significantly lower concomitant saccharide contents than that of S-2 strain as their parent strain.

Among the mutants thus obtained, an example of preferably usable mutant is, for example, mutant MA446 strain (deposited in International Patent Organism Depositary National Institute of Advanced Industrial Science and Technology under the accession number of FERM BP-11250).

Films with a relatively high rupture strength of piercing can be obtained by using the particulate composition containing pullulan of the present invention, which is an inexpensive product produced without employing any complicated purification step of solvent precipitation or the like. Thus, the particulate composition has the merit that it is more extensively used when formed into, for example, a pullulan film. According to the process for producing the particulate composition containing pullulan of the present invention, there is obtained the merit that the above-identified particulate composition containing pullulan with the above-mentioned advantageous merit can be obtained easily and cheaply without employing any complicated purification step of solvent precipitation or the like.

The present invention solves the above object by providing a shaped product containing pullulan produced by using at least partly the particulate composition containing pullulan of the present invention as a material. As described above, although the particulate composition containing pullulan of the present invention is so called "General-Purpose Industrial Product" produced without employing any purification step for pullulan, such as solvent precipitation, it has an advantageous feature that it imparts a relatively high rupture strength of piercing when formed into a film. Further, the particulate composition containing pullulan of the present invention has the following features so that it can be used as a pharmaceutical additive in pharmaceuticals, quasi-drugs, cosmetics, etc., which require a constant disposition of effective ingredients; the concomitant saccharide content is extremely as low as 3% by weight or lower, the saccharide composition is almost stably retained independently of its production lot because it has a relatively high purity of pullulan, and the rupture strength and the physical properties such as solubility and adhesive power less vary and less differ. Examples of the forms used in pharmaceuticals, etc., the particulate composition can be formed into films, as well as, for example, sheets, tablets, granules, and fibers, or coating agents, sugar coating agents, diluents/fillers/adjuvants, adhesive agents, and solid preparations such as a solid preparation to be reconstituted upon use directed for use in liquids. Accordingly, the particulate composition can provide shaped products which have constant and persistent properties required for pharmaceuticals, quasi-drugs, cosmetics, etc. Particularly, when used in pharmaceuticals, the particulate composition containing pullulan of the present invention can provide products that each have lesser dispersion in terms of the aspects of physical properties such as dissolution rate, integration rate, and rupture strength; and it has a lesser fear of affecting the disposition of effective ingredients due to dissolution or disintegration thereof. Thus, the particulate composition can be used as a pharmaceutical additive. The particulate composition containing pullulan of the present invention can be also used as a material for solid medicines in the form of a solid preparation to be reconstituted upon use directed for use in injections, etc., by applying thereunto a conventional method in general to make pyrogen free.

Brief Description of Drawings

FIG. 1 is an elution pattern of an aqueous solution of the particulate composition containing pullulan of the present invention when subjected to gel permeation chromatography.
FIG. 2 is an elution pattern of an aqueous solution of "Food Additive Pullulan" when subjected to gel permeation chromatography.
FIG. 3 is an elution pattern of an aqueous solution of a concomitant saccharide fraction dissolvable in 75% by volume of methanol in water of the particulate composition containing pullulan of the present invention, when subjected to gel permeation chromatography.
FIG. 4 is an elution pattern of an aqueous solution of a concomitant saccharide fraction dissolvable in 75% by volume of methanol in water of "Food Additive Pullulan", when subjected to gel permeation chromatography.
FIG. 5 is a diffraction pattern of X-ray small angle scattering analysis in the range of scattering angle $2\theta$ of 0.001 to 0.1° on a radiation light of a film formed with the particulate composition containing pullulan of the present invention. FIG. 6 is a diffraction pattern of X-ray small angle scattering analysis in the range of scattering angle 2θ of 0.01 to 2° on a radiation light of a film formed with the particulate composition containing pullulan of the present invention.

Best Mode for Carrying Out the Invention

The particulate composition containing pullulan of the present invention is characterized in that it is produced by culturing a mutant of a microorganism of the species *Aureobasidium pullulans* in a culture medium containing glucose and maltose as carbon sources without employing a step of removing concomitant saccharides, it consists of a pullulan fraction and a concomitant fraction which are respectively insoluble and soluble in 75% by volume of methanol in water; it has a ratio of 3% by weight or lower of concomitant saccharides against the content of total saccharides contained in the whole particulate composition; and it contains mannitol. The term "75% by volume of methanol in water" means a mixture that contains water and methanol in a volume ratio of 1:3.

As long as mutants of a microorganism of the species *Aureobasidium pullulans* can produce the particulate composition containing pullulan of the present invention without employing a step of removing concomitant saccharides from the resulting cultures, any mutants can be used in the present invention. Examples of parent strains usable in the present invention include *Pullularia* sp. S-2 strain, a pullulan producing microorganism, disclosed by Seinosuke UEDA in "Kogyo-Kagaku-Zasshi" (The Journal of Chemical Industry), Vol. 67, pp. 757-760, 1964. At present, the microorganisms of the genus *Pullularia* are classified into those of the genus *Aureobasidium* and there has only been known one species, *Aureobasidium pullulans.* Since S-2 strain produces pullulan and, as described later, it has the cultural characteristics that well coincide with those of *Aureobasidium pullulans*, it has been identified as a microorganism of the species *Aureobasidium pullulans* and deposited on June 28, 2010 in International Patent Organism Depositary National Institute of Advanced Industrial Science and Technology, AIST Tsukuba Center 6, 1-1, Higashi 1-chome Tsukuba-shi, Ibaraki-ken, Japan, under the accession number of FERM BP-11261. To obtain mutants of a microorganism of the species *Aureobasidium pullulans* usable in the present invention, the above-identified S-2 strain is treated with mutation treatment conducted conventionally in the art such as exposure to ultraviolet rays and agent treatments with mutation-inducible agents, for example, as disclosed in Sumita SUGIYAMA et al., "Shin-Pan-Biseibutsu-Kagaku-Jikken-Ho", pp. 126-133, Kodansha Scientific Ltd., Tokyo, Japan, March 20, 1999; the resulting mutants are allowed to culture in a culture medium containing glucose and maltose as carbon sources, followed by screening the desired mutants with an index of the content of concomitant saccharides contained in the resulting cultures; and the ones, which the content of concomitant saccharides is significantly lower than that of S-2 strain as a parent strain, should be selected. The later-described Experiment 1 describes in detail an example of such screening. Examples of the desirable mutants usable in the present invention are those which the content of concomitant saccharides is lower than that contained in the resulting culture medium when they are cultured in a selection culture medium containing glucose and maltose as carbon sources, and more preferably, those which the content of concomitant saccharides is not higher than 50% of that of the culture of S-2 strain as a parent strain.

As described above, although the particulate composition containing pullulan of the present invention should never be restricted to those which are produced from the culture of a specific mutant, concrete examples of preferable mutants that can produce the particulate composition containing pullulan of the present invention include *Aureobasidium pullulans* MA446 strain obtained by mutating the above *Aureobasidium pullulans* S-2 strain. Similarly as in S-2 strain as a parent strain, the mutant MA446 strain produces pullulan and has cultural characteristics well coincided with those of *Aureobasidium pullulans*, therefore, it was identified as a microorganisms belonging to *Aureobasidium pullulans* and has been deposited on April 30, 2010 in International Patent Organism Depositary National Institute of Advanced Industrial Science and Technology, AIST Tsukuba Center 6, 1-1, Higashi 1-chome Tsukuba-shi, Ibaraki-ken, Japan, under the accession number of FERM BP-11250.

For comparison, S-2 strain as a parent strain and mutant MA446 strain have the following cultural characteristics:

<Cultural characteristics of S-2 strain and mutant MA446 strain>

They grow aerobically at 27°C on potato-dextrose agar medium (a potato exudate powder 4.0 g, glucose 20.0 g, agar 15.0 g, refined water 1,000 ml, pH 5.6) and produce a dark color pigment. There appears neither fruit body nor moving in a deformed shape. Sexual reproduction is not observed but a septal wall is observed. After about four days of growth, they form conidiophores in the form of a unisexual aciniform budding and form conidiophores in the form of a budding at the edge or the intermediate of a linear hypha. Matured conidiophores exist in the form of a monospore, have no color, and have smooth surface.

Any culture media can be used in culturing mutants as long as they can produce the particulate composition containing pullulan of the present invention basically without employing a step of removing concomitant saccharides from the resulting cultures which contain lesser amounts of concomitant saccharides, and further those which appropriately contain carbon sources, nitrogen sources, organic nutrition sources, inorganic substances, etc., similarly as those which are used in culturing a microorganism of the species *Aureobasidium pullulans* for producing pullulan.

Examples of the carbon sources include saccharide mixtures containing glucose and maltose, preferably, saccharides which contain glucose and maltose having a dextrose equivalent (abbreviated as "DE", hereinafter) of 50 to 90, and more preferably, saccharides which contain glucose and maltose in respective amounts of at least 10% by weight, d.s.b. In the case of lacking either or both of glucose and maltose, the content of concomitant saccharides does not become so lowered, resulting in a difficulty of producing the particulate composition containing pullulan of the present invention. Also, it is undesirable that, in the case of using saccharides, as carbon sources, with a DE of less than 50 or over 90, the yield of pullulan decreases to lower the content of pullulan in the resulting particulate composition containing pullulan and to relatively increase the content of concomitant saccharides. The concentration of carbon sources in culture media is desirable to be 5 to 20 w/v %.

Examples of nitrogen sources include one or more ingredients selected from inorganic nitrogen sources such as ammonium salts and nitrates; and organic nitrogen sources such as glutamates, peptones, yeast extracts, and corn steep liquor. As other inorganic substances, phosphates, magnesium salts, and iron salts can be appropriately used. The conditions of culturing microorganisms are desirably conducted aerobically while stirring or shaking under aeration conditions. As preferable culture temperatures, 27°C is suitable for the growth of *Aureobasidium pullulans.* As culturing time, it is preferably be conducted up to almost reach the maximum level of pullulan yield after consumption of carbon sources and to reach a roughly constant level of concomitant saccharides after decrease thereof. If necessary, to increase the yield of pullulan or to produce pullulan with a desired molecular weight, continuous culture can be employed by adding appropriate pH controlling agents to the culture media or controlling the dilution rate of the culture liquids by extracting samples therefrom intervally or continuously and by appropriately supplying or supplementing thereunto a fresh preparation of a nutrient culture media.

Culture supernatants containing pullulan can be obtained from the resulting cultures by removing cells from the cultures with conventionally used appropriate methods such as centrifugation and filtration. The particulate composition containing pullulan of the present invention can be obtained in conventional manner by decoloring, desalting, concentrating, drying, and pulverizing the culture supernatants.

In general, filtration with a powdered activated charcoal can be employed as decoloration treatment. Cation-exchange resins and anion-exchange resins are usually used in desalting treatment. Examples of cation-exchange resins include commercialized cation-exchange resins such as "DIAION PK218" (Mitsubishi Chemical Co., Ltd., Tokyo, Japan), "DIAION SK-1B" (Mitsubishi Chemical Co., Ltd., Tokyo, Japan) ; and examples of anion-exchange resins include commercialized anion-exchange resins such as "DIAION WA30" (Mitsubishi Chemical Co., Ltd., Tokyo, Japan), and "AMBERLITE IRA411" (Japan Organo Co., Ltd., Tokyo, Japan).

Drying and pulverization of the culture supernatants can be conducted based on conventional methods . For example, after drying, the resulting blocks can be pulverized into particulate compositions or the culture supernatants can be simultaneously dried and pulverized by a spray-drying method.

The particulate composition containing pullulan of the present invention thus obtained contains 3% by weight or lower of concomitant saccharides against the content of total saccharides contained in the whole particulate composition, in other words, it stably contains pullulan in an amount of 97% by weight or higher, and more preferably, the composition contains 1% by weight or lower of concomitant saccharides against the content of total saccharides contained in the whole particulate composition, in other words, it stably contains pullulan in an amount of 99% by weight or more and contains mannitol.

In this connection, the concomitant saccharides in the concomitant saccharide fraction contain saccharides with a glucose polymerization degree of 3 to 90 in the particulate composition containing pullulan of the present invention. The term "saccharides with a glucose polymerization degree of 3 to 90" as referred to as in the present invention concretely means, as described in the later discussed Experiment 4, ingredients that are detected in the range of those with a molecular weight corresponding to a glucose polymerization degree of 3 to 90, when a concomitant saccharide fraction dissolvable in 75% by volume of methanol in water of the particulate composition is subjected to gel permeation chromatography (abbreviated as "GPC", hereinafter); namely, those which are detected in the range of molecular weight of 500 to 15, 000 daltons in view of the fact that the average molecular weight determined on GPC has a constant error.

In the particulate composition containing pullulan of the present invention, the content of saccharides with a glucose polymerization degree of 3 to 90 is preferably 2% by weight or lower of the content of total saccharides contained in the whole particulate composition. When the content of saccharides with a glucose polymerization degree of 3 to 90 exceeds 2% by weight, it is not desirable because a prescribed rupture strength of piercing may not be obtained when formed into a film. In this connection, the above saccharides with a glucose polymerization degree of 3 to 90 is deemed to be those which have basically a pullulan-like structure wherein maltotriose molecules are coupled in an $\alpha$-1,6 linkage fashion due to the fact that they form both maltotriose as a main ingredient and maltotetraose in a relatively small amount when subjected to the action of pullulanase. The saccharides, however, are those which have a relatively low molecular weight and dissolve in 75% by volume of methanol in water, though they have a pullulan-like structure.

As described above, varying depending on the types of carbon sources used in culture media, the culture of a microorganism of the species *Aureobasidium pullulans* usually contains mannitol as a metabolite thereof. Mannitol is dissolvable in 75% by volume of methanol in water and it is contained in the particulate composition containing pullulan of the present invention, unless the concomitant saccharides are removed from the culture by solvent precipitation or the like. Thus, the fact that mannitol is present in the particulate composition containing pullulan means that the particulate composition is the one produced without employing a step of removing concomitant saccharides by solvent precipitation or the like. As described above, mannitol is not detected by the anthrone-sulfuric acid method; however, the mannitol in the particulate composition can be determined on the later described high-performance liquid chromatography (HPLC) analysis used in Experiment4. The content of mannitol in the particulate composition containing pullulan of the present invention is usually 0.04% by weight or higher, d.s.b., and it never exceeds 2% by weight.

Although the molecular weight of pullulan as the major ingredient of the particulate composition containing pullulan of the present invention should not specifically be restricted to specific ones as long as such pullulan imparts a desired rupture strength of piercing when formed into a film, it is desirable to have a weight-average molecular weight, determined on GPC (designated as "Mw", hereinafter), of about 50,000 to 1,000,000 daltons, more preferably, about 50,000 to 500,000 daltons. When the molecular weight of pullulan falls below Mw 50,000 daltons, it is not preferable because such pullulan is hardly to be formed into a film. While, when the molecular weight of pullulan exceeds Mw 1,000,000 daltons, the viscosity of an aqueous solution of such pullulan may become too high to be used easily as a problem.

In a particulate composition containing pullulan produced from a culture of a microorganism of the species *Aureobasidium pullulans* through the steps of decoloring, desalting, concentrating, drying, and pulverizing without employing any removing step for removing concomitant saccharides by solvent precipitation or the like, the ratio of Mw against the number average molecular weight of pullulan (designated as "Mn", hereinafter) is usually about 10 to 70 (Mw/Mn) . In the particulate composition containing pullulan of the present invention, the ratio of Mw/Mn should not specifically be restricted to specific ones as long as the desired rupture strength of piercing is obtained, when formed into a film; however, preferable ratios are 100 or lower, more preferably, those in the range of about 10 to about 70. When the ratio of Mw/Mn exceeds 100, namely when the particulate composition has a wide range of molecular weight distribution of pullulan, the membrane formability of the particulate composition is poor and, when formed into a film, any film with a stable, high rupture strength of piercing could not easily be obtained.

The particulate composition containing pullulan of the present invention has white appearance and satisfactory free-flowing-ability, and exhibits a preferable solubility in water similarly as in conventional particulate composition containing pullulan. Depending on use, the particulate composition containing pullulan of the present invention can be used after mixing with one or more ingredients, used in the fields of food products, pharmaceuticals, quasi-drugs, and cosmetics in general, selected from the group consisting of other materials, for example, polysaccharides other than pullulan, bulking agents, excipients/adjuvants, fillers, viscosity-imparting agents, surfactants, foaming agents, antifoam agents, pH-controlling agents, stabilizers, flame retardants, mold release agents, antiseptics, colors, flavors, nutrients, preferences including tobaccos, taste-imparting agents, medicines, and physiologically active substances. When formed into a film as a concrete example of a shaped product made by the particulate composition containing pullulan of the present invention, the film has a satisfactory rupture strength of piercing, has a satisfactory solubility similarly as in conventional particulate compositions containing pullulan, and has an advantageous durability in use. Thus, the particulate composition containing pullulan of the present invention can be used as a material for films, sheets, and coatings used in the fields of food products, pharmaceuticals, cosmetics, etc. In the case of forming the particulate composition containing pullulan of the present invention into a film or the like, there can be used surfactants containing fatty acid esters of saccharides, such as sugar fatty acid ester as a release agent.

As described above, the particulate composition containing pullulan of the present invention has both a satisfactory solubility in water and a satisfactory high strength when formed into films or the like; has a high purity of pullulan in that the content of concomitant saccharides to the total saccharides is 3% by weight or lower and the pullulan content is 97% by weight or higher; has a roughly constant saccharide composition, and has a stably constant range of molecular weight distribution of pullulan *per se.* Therefore, when used in shaped products, the particulate composition is expected to impart a remarkably-constant fixed strength, dissolution rate, and disintegration rate to the shaped products. Accordingly, the particulate composition containing pullulan of the present invention can be used in food products and also used as a pharmaceutical additive in pharmaceuticals, quasi-drugs, cosmetics, etc., where the disposition of effective ingredients thereof is required to be remarkably-consistent. The particulate composition can be used in films, as well as shaped products such as fibers for sheets, gauzes, surgical strings, etc.; it can be used as excipients/adjuvants/fillers, adhesives or coating agents in preparing tablets and granules. Further, the particulate composition can be prepared into solid medicines in the form of a solid preparation to be reconstituted upon use for use in liquid preparations. These cosmetics, pharmaceuticals, and quasi-drugs thus obtained have no disparity but have stable, constant, and consistent quality independently of their production lots. In particular, in pharmaceuticals, there is no fear of affecting on the disposition of effective ingredients due to the variability in solubility and disintegrability.

In addition to the particulate composition containing pullulan of the present invention, ingredients widely used in respective fields can be appropriately added to shaped products produced by using the particulate composition at least partly as a material thereof. When the above shaped products are cosmetics or their intermediates, they can be formed into, for example, packs, masks, bath salts, or cachou films. One or more of the following ingredients can be appropriately added alone or in combination to the above shaped products: Antiseptics such as paraoxybenzoic acid, benzalkonium chloride, and pentanediol; skin whitening agents such as albutin, ellagic acid, tetrahydrocurcuminoid, and vitamin P; anti-infrrammatories such as glycyrrhizic acid and glycyrrhiza extract; cell activators such as lactoferrin, chondroitin sulfate, hyaluronic acid, KANKOSO-101, and KANKOSO-301; humectants such as elastin, keratin, urea, and ceramide; oil-based medicines such as squalane, petrolatum, and tri-2-ethyl hexanoic acid cetyl; and water-soluble high molecules such as carrageenan, carboxymethyl cellulose, locust bean gum, and carboxy vinyl polymer; and alcohols such as 1,3-butylene glycol, polyethylene glycol, propylene glycol, sorbitol, and maltitol.

In the case that the above shaped products are pharmaceuticals, quasi-drugs, or intermediates thereof, they can be formulated into granules, tablets, sugar-coated tablets, etc. One or more of the following ingredients can be appropriately added alone or in combination to the above formulated products: Immunosuppresants such as azathioprine, cyclosporine, cyclophosphamide, methotrexate, tacrolimus hydrate, and busulfan; anticancer agents such as capecitabine, rituximab, trastuzumab, bevacizumab, docetaxel, imatinib mesylate, 5-fluorouracil, anastrozole, taxol, tamoxifen, docetaxel, and hydroxycarbamide; anti-viral agents such as abacavir sulfate, zalcitabine, didanosine, famciclovir, and ribavirin; antibiotics such as amoxicillin, talampicillin, cefixime, sulfamethizole, levofloxacin hydrate, cefcapene pivoxil hydrochloride hydrate, cefditoren pivoxil, and clarithromycin; antipyretic-analgesics such as acetaminophen, aspirin, ethenzamide, and methyl salicylate; steroids such as prednisolone, dexamethasone, and betamethasone; proteins or pepetides such as interferon-$\alpha$, interferon-$\beta$, insulin, oxytocin, and somatropin; biological drugs such as BCG vaccine, Japanese encephalitis vaccine, measles vaccine, polio vaccine, vaccine, tetanus toxoid, habu antitoxin, and human immunoglobulin; vitamin preparations and derivatives thereof such as retinol, thiamine, riboflavin, pyridoxine, cyanocobalamin, L-ascorbic acid, carotenoid, ergosterol, tocopherol, biotin, calcitonin, Coenzyme Q, $\alpha$-lipoic acid, nicotinic acid, menaquinone, ubiquinone, and pyrroloquinone quinoline; and crude drug extracts such as Korean ginseng extract, aloe extract, propolis extract, glycyrrhiza extract, cinnamon extract, and *Swertia japonica* extract.

Within the scope of the present invention, where the effects and functions of the present invention will exert, and in order to improve the flexibility and strength, the above-identified shapedproduct such as cosmetics, pharmaceuticals, quasi-drugs, and intermediates thereof can be, if necessary, arbitrarily admixed with other high molecular substances, appropriate plasticizers, or fillers/excipients/adjuvants, etc., which are generally used in the fields of cosmetics, pharmaceuticals, and quasi-drugs, along with the particulate composition containing pullulan of the present invention. In the shaped products that are mainly composed of other fillers/excipients/adjuvants, the particulate composition containing pullulan of the present invention can be used as an adhesive. Examples of the high molecular substances include polysaccharides or derivatives thereof such as carrageenan, xanthan gum, carboxymethyl cellulose, cellulose, hemicelluloses, gum arabic, guar gum, carrageenan, pectin, agarose, dextrin, amylose, and starch containing processed starch; and proteins such as gelatin and casein. Examples of the plasticizers include saccharides such as maltitol, mannitol, maltitol, sucrose, maltose, lactose, $\alpha,\alpha$-trehalose, $\alpha,\beta$-trehalose, gum arabic, corn starch, and crystalline cellulose; and inorganic substances such as aluminum hydroxide, calcium hydroxide, magnesium hydroxide, barium hydroxide, calcium sulfate, calcium sulfite, calcium carbonate, silica, calcium silicate, basic magnesium carbonate, kaolin, and talc. In particular, $\alpha,\alpha$-trehalose can be advantageously used because it inhibits the deterioration of effective ingredients due to their oxidation decomposition and has functions of stably retaining the activity of the effective ingredients.

The present invention will be explained with reference to the following Experiments.

<Experiment 1: Obtension of mutant of pullulan producing microorganism>

Conidiophores of *Aureobasidium pullulans* S-2 strain were irradiated with ultraviolet rays to induce mutation and inoculated to potato-dextrose agar plate, followed by selecting as a mutant a colony that had grown at 27°C. The separated colony and S-2 strain as a parent strain were respectively inoculated to a liquid selection medium (pH 7.0) containing 10.0 w/v %, d.s.b., of a glucose syrup resolved by acid (DE 48, containing, on a dry solidbasis, about 27% glucose and about 17% maltose, commercialized by Hayashibara Shoji, Co., Ltd., Okayama, Japan), 0.2 w/v % dibasic potassium phosphate, 0.2 w/v % peptone, 0.2 w/v % sodium chloride, 0.04 w/v % magnesium sulfate heptahydrate, and 0.001 w/v % ferrous sulfate heptahydrate, and cultured under shaking conditions at 27°C for three days. The culture supernatants, which had been obtained by centrifuging each resulting culture to remove cells, were diluted with water by 10 times. To each portion of which was added 3-times volume of methanol (a special grade reagent, commercialized by Wako Pure Chemical Industries, Ltd., Tokyo, Japan) to make into a 75% aqueous methanol solution, and the resulting mixture was well mixed to precipitate pullulan, followed by centrifuging the resulting mixture to obtain a supernatant which was then diluted by 10-times with water for use as a test solution for assaying concomitant saccharides. The test solution corresponds to a dilute that had been prepared by diluting each supernatant by 400-times. A 0.01 w/v % aqueous glucose solution was provided as a standard solution and water was used as a blank control, and each test solution was subjected to color reaction based on the anthrone-sulfuric acid method in accordance with the description of "Japanese Standard of Food Additives", 8th edition, pp. 572-573, 2007, published by Japan Food Additives Association, followed by measuring the absorbance of the resultant mixture at a wavelength of 620 nm by using a spectrophotometer and determining the concentration (%) of concomitant saccharides of each supernatant based on the following Equation 1. While, the dilute of each supernatant that had been diluted by 10-times was further diluted with water by 100-times for use as a test solution for measuring the total saccharides. Similarly as above, the test solution was induced color reaction based on the anthrone-sulfuric acid method and measured for its absorbance at a wavelength of 620 nm, followed by determining the concentration (%) of total saccharides of each supernatant by using the following Equation 2. Pullulan concentration (%) was determined by subtracting the concomitant saccharide concentration from the total saccharide concentration by using the following Equation 3.

Equation 1:

$$\text{Concentration (\%) of concomitant saccharides} = [\{(Ak-A_0) \times 400\}/\{(As-A_0) \times 100\}] \times 100$$

Ak: Absorbance of test solution for measurement of concomitant saccharides;

As: Absorbance of standard solution

$A_0$: Absorbance of water (control)

Equation 2:

$$\text{Concentration (\%) of total saccharides} = [\{(Az-A_0) \times 1000\}/\{(As-A_0) \times 100\}] \times 100$$

Az: Absorbance of test solution for measurement of total saccharides;

As, and $A_0$: Same as in Equation 1

Equation 3:

$$\text{Pullulan concentration (\%)} = (\text{Concentration (\%) of total saccharides}) - (\text{Concentration (\%) of concomitant saccharides})$$

The concentration of total saccharides in culture medium prior to cell inoculation was subjected to the anthrone-sulfuric acid method for determining the concentration (%) of carbon sources, followed by determining, as the yield (%) of concomitant saccharides and the yield (%) of pullulan against saccharides, the concentration (%) of concomitant saccharides and the concentration (%) of pullulan against the concentration (%) of carbon sources in the culture medium, determined based on by the following Equations 4 and 5, respectively.

Equation 4:

$$\text{Yield (\%) of concomitant saccharides} = \{(\text{Concentration (\%) of concomitant saccharides})/(\text{Concentration (\%) of carbon sources of culture medium})\} \times 100$$

Equation 5:

Yield (%) of pullulan against saccharides

= {(Concentration (%) of pullulan)/(Concentration

(%) of carbon sources of culture medium)} x 100

As an index of the percentage of concomitant saccharides obtained similarly as in the above, a group of mutants (about one percent of the isolated strains) that had a lesser percentage of concomitant saccharides than those of their parent strains was selected. Among the selected mutants, five strains, which have a particularly low percentage of concomitant saccharides and have at most half the levels of their respective parent strains, are shown in Table 1. As shown in Table 1, these mutants showed almost the same level of the yield of pullulan against saccharides as that of S-2 strain as a parent strain, and the level of each of the strains is as low as 3.0% or lower that is lower than half of that (the concentration of concomitant saccharides: 6.6%) of S-2 strain as a parent strain, while the ratio of "the yield (%) of concomitant saccharides" against "the yield (%) of pullulan against saccharides" for each strain is respectively 0.05 or lower that is less than half of that (0.101) of S-2 strain. These mutants shown in Table 1 have roughly the same level of the yield of pullulan against saccharides as those of their respective parent strains and have a lower yield of concomitant saccharides. Accordingly, even if a particulate composition containing pullulan were produced without employing a purification step such as precipitation separation from their cultures by using solvents, it is greatly expected that there can be obtained a particulate composition containing pullulan with both a lesser amount of concomitant saccharides compared to those of conventional ones and a stable, constant composition. Among the mutants thus obtained, since MA446 stain has the minimum level of the yield of concomitant saccharides and has a lower level of the yield of pullulan against saccharides, it was selected and subjected to the following experiments. Based on the above mutating, culturing and screening methods and by using at least S-2 strain as a parent strain, desired mutants, which can provide the particulate composition containing pullulan of the present invention, can be appropriately obtained without a need of excessive trial and error.

Table 1

| | Mother strain | Mutant | | | | |
|---|---|---|---|---|---|---|
| | S-2 Strain | MA162 Strain | MA446 Strain | MA782 Strain | MA2248 Strain | MA3623 Strain |
| Yield (%) of pullulan against saccharides | 65.2 | 67.5 | 68.3 | 68.9 | 67. 5 | 66.9 |
| Yield (%) of concomitant saccharides | 6.6 | 2.9 | 2.3 | 3.0 | 2.7 | 2.6 |
| (Yield of concomitant saccharides)/ (yield of pullulan against saccharides) | 0.101 | 0.043 | 0.034 | 0.044 | 0.040 | 0.039 |

<Experiment 2: Preparation of concomitant saccharide containing pullulan by using MA446 strain>

<Experiment 2-1: Examination of carbon sources for use in medium for pullulan production>

To produce particulate compositions containing pullulan, MA446 strain was cultured in respective culture media 1 to 5 with carbon sources having different DE values, and examined their properties. The carbon sources used in the culture media 1 to 5 were respectively shown below. The starch syrup prepared by acid saccharification contains, on a dry solid basis, about 25% by weight of glucose and about 16% by weight of maltose.

- Culture medium 1: "Starch syrup" (a starch syrup prepared by acid saccharification, DE of about 42, commercialized by Hayashibara Shoji, Co., Ltd., Okayama, Japan).

- Culture medium 2 : Being prepared by mixing glucose (a special grade reagent commercialized by Wako Pure Chemical Industries, Ltd., Tokyo, Japan) (hereinafter the same as in media 3 to 5), maltose ("MALTOSE HHH", a purity of 99% or higher, commercialized by Hayashibara Biochemical Laboratories, Inc., Okayama, Japan) (same as in media 3 and 4), and a starch syrup ("MALT-RUP®", DE47, a syrup prepared by enzyme saccharification, commercialized by Hayashibara Shoji, Co., Ltd., Okayama, Japan) in a weight ratio of 1:1:3 (DE of about 58 in a mixture form).

- Culture medium 3: Being prepared by mixing glucose, maltose and a starch syrup ("MALT-RUP®", DE 47, a starch syrup prepared by enzyme saccharification) in a weight ratio of 1:1:1, d.s.b., (the mixture has a DE of about 66).

- Culture medium 4: Being prepared by mixing glucose, maltose and starch syrup ("MALT-RUP®", DE 47, a starch syrup prepared by enzyme saccharification) in a weight ratio of 4:1:1, d.s.b., (the mixture has a DE of about 83).

- Culture medium 5: Only glucose (a DE of about 100).

<Experiment 2-2: Preparation of particulate composition containing pullulan>

MA446 Strain, which had been cultured on a potato-dextrose agar slant at 27°C, was subjected to culture under rotatory shaking conditions in a liquid culture medium (pH 7.0) containing 10.0 w/v % sucrose, 0.2 w/v % dibasic potassium phosphate, 0.2 w/v % peptone, 0.2 w/v % sodium chloride, 0.04 w/v % magnesium sulfate heptahydrate, and 0.001 w/v % ferrous sulfate heptahydrate at 27°C at 230 rpm for 48 hours for use as a seed culture.

As pullulan producing media, culture media 1 to 5, containing 10.0 w/v % of any one of the above carbon sources, 0.2 w/v % dibasic potassium phosphate, 0.2 w/v % peptone, 0.2 w/v % sodium chloride, 0.04 w/v % magnesium sulfate heptahydrate, and 0.001 w/v % ferrous sulfate heptahydrate were prepared in a volume of 20 L per one 30 L-culture fermentor, adjusted to pH 7.0 and sterilized at 121°C for 15 min. The resulting cultures were respectively placed in a fermentor in a volume of 1,000 ml, and cultured at 27°C for three days at an aeration rate of 5.0 L/min and a rotation speed of 400 rpm.

Each resulting culture was centrifuged at 8,000 rpm to remove cells to obtain a supernatant, followed by decoloring treatment with filtration using an activated charcoal and desalting treatment with a cation exchange resin, "DIAION SK1B (H⁺-form)", Mitsubishi Chemical Co., Ltd., Tokyo, Japan, and an anion-exchange resin, "DIAION WA30 (OH⁻-form)", Mitsubishi Chemical Co., Ltd., Tokyo, Japan. After filtration for finishing with an activated charcoal, the resulting solutions were concentrated *in vacuo* up to give a concentration of about 25% by weight, d.s.b. The resulting each concentrate was spray dried to obtain a particulate composition containing pullulan. S-2 Strain as a parent strain was cultured in the culture medium 1 similarly as in the above, and treated similarly as above to obtain a particulate composition containing pullulan for use as a control particulate composition containing pullulan. For comparison, S-2 strain was similarly cultured as above in the culture medium 1, and the resulting particulate composition containing pullulan has substantially the same pullulan content and the concomitant saccharide content as those of the above-identified "Food Additive Pullulan", and the above control as the particulate composition containing pullulan corresponds thereunto.

<Experiment 2-3: Measurement of pullulan content and concomitant saccharide in particulate composition containing pullulan>

For the particulate composition containing pullulan prepared with the above culture media 1 to 5 and the control one, they were respectively determined on their concomitant saccharide content and pullulan content by the following methods in accordance with the method disclosed in "Japanese Standard of Food Additives" (8th edition), published by Japan Food Additives Association (JAFA), 2007, pp. 572-573 (see the column of "Pullulan"). 0.8 g each of the particulate compositions containing pullulan was dissolved in 100 ml water for use as a material sample solution. To one milliliter of each material sample solution was added water to give a total volume of 50 ml for use as a standard material solution (where each material sample solution was diluted by 50 times). To one milliliter of each material sample solution was added 0.1 ml of a saturated potassium solution, the resulting mixture was admixed well with three milliliters of methanol, and centrifuged at 4°C and 15,700 g x 10 min to obtain a supernatant for use as a test solution (where each material sample solution was diluted by 4.1 times) . These standard material solutions, sample solutions, and a control water were respectively subjected to the anthrone-sulfuric acid method to induce an expected reaction, followed by measuring their absorbances (At, As, and Ao, respectively) at a wavelength of 620 nm and determining on the following equation both the pullulan content and the concomitant saccharide content, i.e., the concomitant saccharide content which was contained in the concomitant saccharide fraction soluble in 75% by volume of methanol in water and determined based on the anthrone-sulfuric acid method. The measured results are in Table 2. In this experiment, the concomitant saccharide and the pullulan content were determined based on the ratio of the absorbance of each sample solution and each standard material solution, where the ratio was not determined based on D-glucose equivalence by using D-glucose as a standard substance, however, the concomitant saccharide content and the pullulan content were coincided with those determined based on D-glucose equivalence, and each of their contents are expressed with "% by weight".

Equation 6:

Concomitant saccharide content (%)

$$= [\{(At-A_0) \times 4.1\}/\{(As-A_0) \times 50\}] \times 100$$

$$= \{(At-A_0)/(As-A_0)\} \times 8.2$$

At: Absorbance of sample solution
As: Absorbance of standard material solution
$A_0$: Absorbance of water (control)

Equation 7:

Pullulan content (%) = 100 (%) − (Concomitant saccharide content)(%)

Table 2

| Strain | | MA446 strain | | | | | S-2 strain |
|---|---|---|---|---|---|---|---|
| Medium | No. | 1 | 2 | 3 | 4 | 5 | 1 |
| | DE of carbon source | 42 | 58 | 66 | 83 | 100 | 42 |
| Pullulan content (%) | | 93.2 | 97.8 | 99.4 | 99.2 | 95.5 | 91.9 |
| Concomitant saccharide content (%) | | 6.8 | 2.2 | 0.6 | 0.8 | 4.5 | 8.1 |

As shown in Table 2, in the case of culturing MA446 strain in the culture media 2 to 4, the pullulan content in each of the resulting particulate compositions containing pullulan was over 97% by weight that was distinctly higher than 93.2% by weight for S-2 strain as a parent strain. The concomitant saccharide content was 2.2% by weight or lower as below as 3% by weight, which was distinctly lower than 6.8% by weight of that of S-2 strain. While, in the case of culturing with culture medium 1, the concomitant saccharide content was 6.8% by weight that was slightly below the level of that of S-2 strain as a parent strain. When MA446 strain was cultured in the culture medium 5 with DE 100 that consisted of glucose as carbon source, it showed the pullulan content of 95.5% by weight and the concomitant saccharide content of 4.5% by weight, wherein the pullulan content was increased and the concomitant saccharide was decreased compared to those of S-2 strain as a parent strain that was cultured in the culture medium 1. These differences were not so distinct as in the case of the culture media 2 to 4.

Based on these, it was revealed that, when MA446 strain was cultured in a culture medium with a DE of about 50 to about 90, preferably, a DE of about 55 to about 85, a particulate composition containing pullulan with a particularly higher content of pullulan and a distinctly lower content of concomitant saccharides was obtained.

<Experiment 2-4: Strength of films formed with respective particulate compositions containing pullulan>

The particulate composition containing pullulan and the one as a control obtained in Experiment 2-2 were respectively dissolved in deionized water into a 20 w/v % aqueous solution, allowed to stand at 62.5°C and deaerated. An adequate amount of each of the resulting deaerated aqueous solutions was dropped and casted on a vinyl chloride plain plate, dried overnight at 25°C and a relative humidity of 30% to form a film with a thickness of about 100 μm. The formed film was detached from the plate and cut into a circle with a diameter of 19 mm for use as a test sample which was then conditioned for humidity at an ambient temperature and a relatively humidity of 22% for five days before subjecting it to a piercing test for rupture strength.

The piercing test for rupture strength was conducted with "Rheometer CR-500DX" (commercialized by Sun Scientific Co., Ltd., Tokyo, Japan), installed with an adaptor for piercing test with a cross-section area of 1 mm$^2$ in such a manner of perpendicularly pressing the adaptor on the center of the above film, fixed on the apparatus at a velocity of 50 mm/min to cause subsidial fracture, followed by determining the stress for rupture strength of piercing. For films formed respectively from the particulate compositions containing pullulan, 10 sheets of each of the films were determined for rupture strength of piercing and the values were averaged. For each test sample after ruptured, the presence or absence of cracking was macroscopically observed, and the percentage (%) of the test samples with crackings against the tested 10 sheets of the test samples was determined. The results are in Table 3.

Table 3

| Strain | | MA446 Strain | | | | | S-2 Strain |
|---|---|---|---|---|---|---|---|
| Culture medium No. | | 1 | 2 | 3 | 4 | 5 | 1 |
| Film | Rupture strength of piercing (MPa) | 16.6 | 21.1 | 23.9 | 22.2 | 18.1 | 17.1 |
| | Incidence (%) of cracking | 40 | 0 | 0 | 0 | 30 | 50 |

As shown in Table 3, any of the films, which had been formed with the particulate composition containing pullulan that had been produced by culturing MA446 strain in the culture media 2 to 4, showed a rupture strength of piercing of over 20 MPa. The level of which was significantly higher than 17.1 MPa of that formed with the particulate composition containing pullulan as a control, where S-2 strain was cultured in the culture medium 1. The incidence of cracking as in the case of the cultures with the culture media 2 to 4 are all 0% that is extremely low level compared to the percentage of 50% of the culture of S-2 strain with the culture medium 1 as a control. The fact that the incidence of cracking is low means that, even if rupture occurs, such rupture less extends there around and the film, which has 0% of the incidence of cracking and which is formed with the particulate composition produced with any of the cultures of MA446 strain with the culture media 2 to 4, has an advantageous feature in terms of strength.

While, in the case of culturing MA446 strain in the culture medium 1, the rupture strength of piercing is 16.6MPa and the incidence of cracking is 40%, meaning that they are substantially the same as in the case of culturing S-2 strain, as a control, in the culture medium 1. When MA446 strain was cultured in the culture medium 5, there was found a slight improvement compared with that S-2 strain as a control was cultured in the culture medium 1 in both the rupture strength of piercing and the incidence of cracking; however, the difference was not so distinct.

These results well coincide with those in Table 2 where the pullulan content and the concomitant saccharide content were determined, revealing that, in the case of the concomitant saccharide content is 3% by weight or lower, i.e., in the case of the pullulan content is 97% by weight or higher, films, which significantly exceed the rupture strength of piercing of that prepared with the culture of S-2 strain as a control by using the culture medium 1, have a distinctly lower incidence of cracking and have a possibility of obtaining films with an improved strength. In particular, as found in MA446 strain cultured in the culture media 3 and 4, it was revealed that, in the case that the concomitant saccharide content is 1% by weight or lower, i.e., the pullulan content is 99% by weight or higher, more improved rupture strength of piercing can be obtained, and thus it is preferable. All the above-identified films exhibited a satisfactory solubility in water at ambient temperature.

<Experiment 3: Comparison of particulate compositions containing pullulan obtained by different mutants>

In Experiment 2-3, by using the culture media 2, 3 and 4 which provide a particulate composition containing pullulan having a relatively high pullulan content when MA446 strain was cultured therein, S-2 strain and conventional strain of *Aureobasidium pullulans* IFO 6353 strain were cultured similarly as in Experiment 2-3, and the resulting cultures were respectively similarly treated to obtain a particulate composition containing pullulan. The pullulan content and the concomitant saccharide content of the produced particulate compositions were measured similarly as in Experiment 2-3, and similarly as in Experiment 2-4, films prepared with each of the particulate compositions were determined for the rupture strength of piercing and the incidence of cracking. The results are in Table 4. The values for MA446 strain in Table 4 are transcribed from the corresponding columns of Tables 2 and 3.

Table 4

| Culture medium | | 2 | | | 3 | | | 4 | | |
|---|---|---|---|---|---|---|---|---|---|---|
| Strain | | MA446 | S-2 | IFO6353 | MA446 | S-2 | IFO6353 | MA446 | S-2 | IFO6353 |
| Particulate composition containing pullulan | Pullulan content (%) | 97.8 | 93.2 | 90.9 | 99.4 | 92.2 | 93.3 | 99.2 | 93.8 | 94.3 |
| | Concomitant saccharide content (% ) | 2.2 | 6.8 | 9.1 | 0.6 | 7.8 | 6.7 | 0.8 | 6.2 | 5.7 |
| Film | Rupture strength of piercing (MPa) | 21.1 | 16.5 | 17.7 | 23.9 | 16.9 | 17.0 | 22.2 | 18.0 | 17.2 |
| | Incidence (%) of cracking | 0 | 30 | 30 | 0 | 40 | 30 | 0 | 50 | 40 |

As shown in Table 4, any of the particulate compositions containing pullulan prepared from the cultures, obtained by culturing S-2 strain or IFO 6353 strain in the culture media 2 to 4, have a pullulan content of below 95% by weight and a concomitant saccharide content of about 6% by weight or higher, the levels of which are respectively distinctly lower and higher than that prepared from the culture of MA446 strain.

The film formed with the particulate composition containing pullulan obtained by culturing S-2 strain or IFO 6353 strain in the culture media 2 to 4 was ruptured by the stress below the level of 18 MPa on the piercing test for rupture strength. The rupture strength of piercing significantly exceeds the level of over 21 MPa that attained by the film formed with the particulate composition containing pullulan obtained by culturing MA446 stain in the culture media 2 to 4. The film formed with the particulate composition containing pullulan, obtained by culturing S-2 strain or IFO 6353 strain in any of the culture media 2 to 4, shows an incidence of cracking of 30% or higher, meaning that the rupture easily spreads there around when it once occurs.

<Experiment 4: Analysis of particulate composition containing pullulan prepared from the culture of MA446 cultured in the culture medium 3>

As a test powder, the particulate composition containing pullulan, which had been obtained in Experiments 2 to 4 and had the highest rupture strength of piercing, i.e., the one that had been prepared from the culture that had been obtained by culturing MA446 strain in the culture medium 3, were measured for molecular weight distribution on GPC. For the test powder, concomitant saccharides soluble in 75% by volume of methanol in water were prepared therefrom and subjected to GPC analysis. The content of mannitol contained therein was assayed on HPLC. While, "Food Additive Pullulan", as a control powder, a commercially available particulate composition containing pullulan, commercialized by Hayashibara Shoji, Co., Ltd., Okayama, Japan, was similarly measured and analyzed. For the control powder, the pullulan content and the concomitant saccharide content were measured by the anthrone-sulfuric acid method similarly as in Experiment 2-3, and the results are in Table 5. In Table 5, the pullulan content and the concomitant saccharide content were of the test powder transcribed from the corresponding columns of Table 2. The details of measurements are as follows.

<Measurement for average molecular weight and molecular weight distribution>

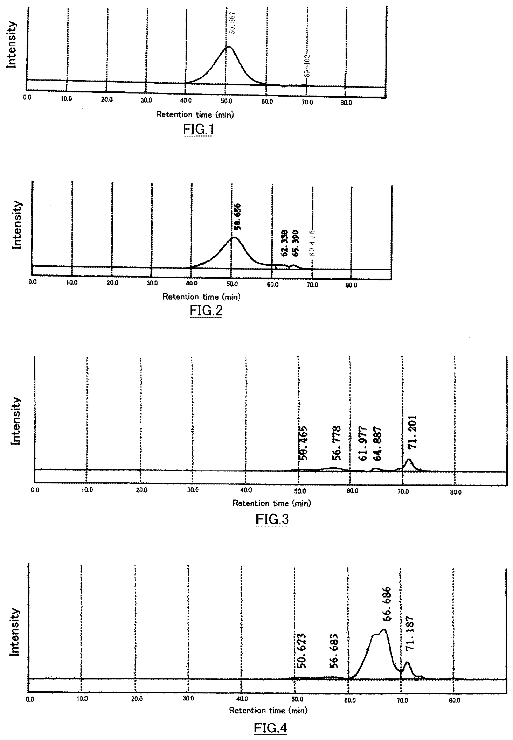

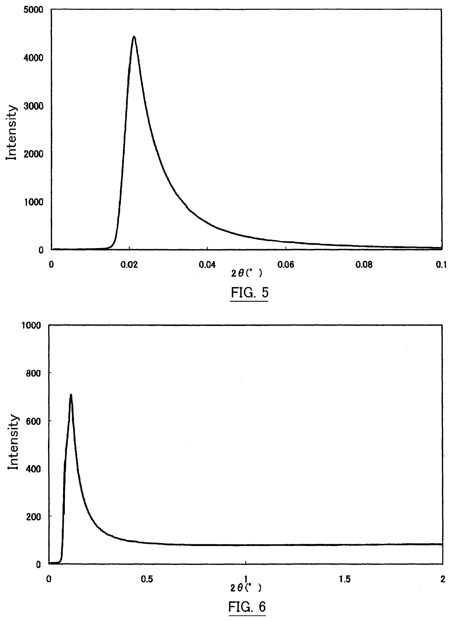

The test powder and the control powder were respectively prepared into a 2 w/v % aqueous solution and admixed with an equal amount of 20 mM phosphate buffer (pH 7.0), and the resulting mixture was membrane filtered for use in GPC analysis. Upon GPC analysis, two columns packed with TSK-GEL $\alpha$-M (7.8 mm$\varphi$ inner diameter x 300 mm length, Tosoh Corporation, Tokyo, Japan) cascaded in series, 10 mM phosphate buffer (pH 7.0) as an eluate, and a differential refractometer as a detector were used, and it was carried out at a temperature of 40°C and a flow rate of 0.3 ml/min. Based on the elution pattern of GPC, the weight-average molecular weight and the number-average molecular weight of pullulan were calculated. The relationship between the molecular weight and the elution time of the test powder and the control powder was determined by using glucose, maltotriose, and pullulan standards for assaying molecular weight, i.e., P-2, P-3, P-5, P-10, P-20, P-50, P-100, P-200, P-400, P-800, P-1600 and P-2500 (commercialized by Showa Denko K.K. Tokyo, Japan) were used. FIGs. 1 and 2 are respectively the GPC elution patterns of the test powder and the control powder. FIG. 5 shows the values of weight-average molecular weight and Mw/Mn.

As shown in FIG. 1, the GPC elution pattern of the test powder consists of a single peak of pullulan that exhibits the top at a retention time of about 50min, and does not substantially contain the ingredients corresponding to the retention times of less than 40 min and over 60 min, meaning that the test powder is of which has a high pullulan purity. While, in the elution pattern of the control powder in FIG. 2, a peak of pullulan that has the top at a retention time of about 50 min is observed, however, several peaks can be observed at the positions corresponding to the retention times of over 60 min, meaning that the control powder contains plenty of concomitant saccharides, other than pullulan, with a lower molecular weight than the pullulan.

<GPC Analysis of concomitant saccharide fraction>

For each powder, concomitant saccharide fractions that are soluble in 75% by volume of methanol in water were prepared as follows: A test powder and a control powder were respectively prepared into a 10 w/v % aqueous solution which was then admixed with 3-times volume of methanol, and the resulting mixture was promptly well mixed, and centrifuged at 4°C and 15,700 g x 10 min. Each supernatant was dried by an evaporator, admixed with refined water to dissolve the contents, and dried. These procedures were repeated twice, followed by removing methanol, redissolving the resultant in refined water, and filtering the solution with a 0.45 $\mu$m membrane filter to obtain a concomitant saccharide fraction soluble in 75% by volume of methanol in water. Each concomitant saccharide fraction was appropriately diluted and subjected to GPC analysis under the same conditions as used in the above molecular weight analysis. FIGs. 3 and 4 are respectively the GPC elution patterns of the concomitant saccharide fractions of the test powder and the control powder.

As found in FIG. 3, several peaks are observed in the concomitant saccharide fraction of the test powder, however, the areas of every peaks are small and negligible in quantity. While as found in FIG. 4, in the concomitant saccharide fraction of the control powder, a broad and large peak was observed at retention times ranging from about 60 min to about 70 min, determining that the peak corresponds to the main ingredients of the concomitant saccharides. The retention times ranging from about 60 min to about 70 min correspond to the molecular weights ranging from about 500 to about 15,000 and roughly correspond to saccharides having a pullulan-like structure, where maltotriose molecules are coupled in series in an $\alpha$-1,6 linkage fashion, with a glucose polymerization degree of about 3 to about 90 (ranging from 1 (molecular weight of 504) to 30 (molecular weight of 14,598) by maltotriose unit).

In FIGs. 3 and 4, the peak observed at a retention time of about 71 min corresponds to mannitol as a metabolite of MA446 strain and S-2 strain used in the formation of pullulan in producing the test powder and the control powder. In this way, the concomitant saccharide fractions of the test powder and the control powder contain mannitol, meaning that both the test powder and the control powder are produced without employing a removing step for removing concomitant saccharides by solvent sedimentation.

<Measurement of the content of saccharides with glucose polymerization degree of 3 to 90>

As described above, mannitol is not assayed on the anthrone-sulfuric acid method and is not contained in concomitant saccharides, however, it is detected as a peak at a retention time of about 71 min on the GPC analysis of concomitant saccharide fraction. The area corresponding to mannitol, which is subtracted from the whole area of elution fractions obtained in FIGs. 3 and 4, is regarded as the area of concomitant saccharides measured on the anthrone sulfuric acid method. Then, the percentage of the area of a peak, corresponding to the saccharides with a glucose polymerization degree of 3 to 90, against the above-identified peak area is determined and shown in Table 5 as the content of saccharides with a glucose polymerization degree (DP) of 3 to 90 ("saccharide content (%) with a DP of 3 to 90 in concomitant saccharides") measured on the anthrone sulfuric acid method. The above "saccharide content (%) with a DP of 3 to 90 in concomitant saccharides" is multiplied by the concomitant saccharide content (%) against the total saccharide content contained in the whole powder that is alternatively determined on the anthrone-sulfuric acid method, and the resultant value is regarded as "saccharide content (%) with a DP of 3 to 90 inparticulate composition containing pullulan" and is shown in Table 5 in parallel. In Table 5, the value of "saccharide content (%) with a DP of 3 to 90 in particulate composition containing pullulan" is calculated by multiplying "content (%) of concomitant saccharides with a DP of 3 to 90", obtained by GPC analysis as shown in Table 5, by "content (%) of concomitant saccharides" obtained by the anthrone-sulfuric acid method in Table 5.

<Measurement of mannitol content in particulate composition containing pullulan>

The test powder and the control powder were analyzed on HPLC to determine the mannitol contents in the particulate compositions containing pullulan. The HPLC analysis was conducted by using a column of MCI GEL CK08EC (8 mm$\varphi$ inner diameter x 300 mm length, Mitsubishi Chemical Co., Ltd., Tokyo, Japan), refined water as an eluant, and a differential refractometer as a detector, and the elution conditions were 75°C and an eluant flow-rate of 0.6 ml/min. By using mannitol with the known concentration as a standard, the mannitol content was determined, on a dry solid basis, based on the peak area in the particulate composition containing pullulan. The results are in Table 5.

Table 5

| Particulate composition containing pullulan | Test powder | Control powder |
|---|---|---|
| Content (%) of concomitant saccharides | 0.6 | 7.7 |
| Content (%) of pullulan | 99.4 | 92.3 |
| Weight-average molecular weight | 417,000 | 374,000 |
| Mw/Mn | 32.4 | 35.0 |
| Content (%) of saccharides with a DP of 3 to 90 in concomitant saccharides | 24.2 | 85.1 |
| Content (%) of saccharides with a DP of 3 to 90 inparticulate composition containing pullulan | 0.1 | 6.5 |
| Content (%) of mannitol in particulate composition containing pullulan | 0.35 | 0.51 |

As shown in Table 5, the test powder produced from the culture of MA446 strain showed no great difference in both the average-molecular weight and the molecular weight distribution compared to those of the control powder. In the test powder, the content of saccharides with a glucose polymerization degree (DP) of 3 to 90 in the concomitant saccharides was 24.2% by weight (the content of saccharides with a DP of 3 to 90 in the particulate composition containing pullulan is 0.1% by weight), which was lower than 85.1% by weight for the control powder (the content of saccharides with a DP of 3 to 90 in the particulate composition containing pullulan is 6.5% by weight) . The content of mannitol in particulate composition containing pullulan of the test powder and the control powder were respectively 0.35% by weight and 0.51% by weight. In the test powder with a low content of concomitant saccharides, the content of saccharides with a DP of 3 to 90 in concomitant saccharides is also low, compared to the control powder.

<Experiment 5: Relationship between the strength of film and the content of concomitant saccharides in particulate composition containing pullulan>

The test powder and the control powder, which had been used in Experiment 4, were mixed in a ratio shown in Table 6 to prepare test samples 1 to 10 with different contents of concomitant saccharides. Table 6 shows the content of pullulan, the content of concomitant saccharides, and the content of saccharides with a glucose polymerization degree (DP) of 3 to 90. By using test samples, films were formed and compared their strength. The formation of the films and the measurement for rupture strength of piercing of the films were conducted in such a manner similarly as in Experiment 2-4. The results are in Table 7.

Table 6

| | Percentage of particulate composition containing pullulan | | Content (%) of pullulan | Content (%) of concomi-tant saccha-rides | Content (%) of saccharides with DP 3 to 90 | |
| | MA446 | Control | | | In concomitant saccharides | In particulate composition containing pullulan |
|---|---|---|---|---|---|---|
| Test sample 1 | 100 | 0 | 99.4 | 0.6 | 24.2 | 0.1 |
| Test sample 2 | 90 | 10 | 98.7 | 1.3 | 61.3 | 0.8 |
| Test sample 3 | 80 | 20 | 98.0 | 2.0 | 71.3 | 1.4 |
| Test sample 4 | 70 | 30 | 97.3 | 2.7 | 76.2 | 2.0 |
| Test sample 5 | 60 | 40 | 96.6 | 3.4 | 79.0 | 2.7 |
| Test sample 6 | 50 | 50 | 95.8 | 4.2 | 80.9 | 3.3 |
| Test sample 7 | 40 | 60 | 95.1 | 4.9 | 82.2 | 4.0 |
| Test sample 8 | 30 | 70 | 94.4 | 5.6 | 83.2 | 4.6 |

(continued)

| | Percentage of particulate composition containing pullulan | | Content (%) of pullulan | Content (%) of concomi-tant saccha-rides | Content (%) of saccharides with DP 3 to 90 | |
| | MA446 | Control | | | In concomitant saccharides | In particulate composition containing pullulan |
|---|---|---|---|---|---|---|
| Test sam ple 9 | 20 | 80 | 93.7 | 6.3 | 83.9 | 5.2 |
| Test sample 10 | 0 | 100 | 92.3 | 7.7 | 85.1 | 6.5 |

Table 7

| Test sample | 1 | 2 | 3 | 4 | 5 | 6 | 7 | 8 | 9 | 10 |
|---|---|---|---|---|---|---|---|---|---|---|
| Rupture strength of piercing (MPa) | 20.9 | 20.5 | 20.2 | 20.1 | 19.3 | 18.9 | 18.4 | 18.0 | 17.7 | 17.3 |
| Incidence (%) of cracking | 0 | 0 | 0 | 0 | 20 | 40 | 40 | 50 | 50 | 60 |

As shown in Tables 6 and 7, the films, which had been formed with the test samples 1 to 4 having a pullulan content of 97% by weight or higher and the concomitant saccharide content of 3% by weight or lower, showed a higher rupture strength of piercing compared to the film which had been formed with the test sample 10 consisting of the control powder, and did not cause rupture even when subjected to a stress of 20 MPa. While in the case of the test samples 5 to 9 with a pullulan content of less than 97% by weight and a saccharide concomitant content of over 3% by weight, the films formed therewith were ruptured when subjected to a stress of below 20 MPa. These results indicate that the rupture strength of piercing tends to decrease as the increase of both the content of concomitant saccharides and the content of saccharides with a glucose polymerization degree of 3 to 90 as the main ingredients of the concomitant saccharides. In the test samples 1 to 4 with a pullulan content of 97% by weight or higher and a concomitant saccharide content of 3% by weight or lower, no crack was induced when the films were ruptured, while in the case of the test samples 5 to 10 with a concomitant saccharide content of 3% by weight or higher, crackings were observed around the ruptured parts, and there was found a tendency of increasing the frequency of causing cracks as the increase of concomitant saccharide content. In test sample 4 with a concomitant saccharide content of 2.7% by weight that is lower than 3% by weight, the content of saccharides with a glucose polymerization degree of 3 to 90 is 2% by weight or lower. To attain a higher rupture strength of piercing and a lower incidence of cracking, it was judged that the content of saccharides with a glucose polymerization degree of 3 to 90 should preferably be a level of 2% by weight or lower.

<Experiment 6: Analysis of pullulan film on radiation light>

According to the method in Experiment 2, two grams of a particulate composition containing pullulan, which had been obtained by culturing MA446 strain in the culture medium 3 was dissolved by heating in 18 g of deionized water, and the resulting solution was casted on a plain plate, dried at 30°C, and allowed to stand at a relative humidity of 22% for 24 hours to form a film with a thickness of about 500 $\mu$m. The film thus obtained was subjected to small-angle X-ray scattering (SAXS) analysis which uses a synchrotron radiation as a radiation source. The measurement was conducted by using "Beam line of Hyogo Prefecture (BL08B2)" placed at "SPring-8", a large scale radiation facility, 1-1-1Koto, Sayo-cho, Sayo, Hyogo, Japan under the following conditions. In particular, to monitor scattering with a scattering angle of 0.1° or lower, it was measured under a condition of longer camera length (the following <SAXS (long)>).

<SAXS (long)>

Wavelength: 1.50 Å

Camera length: 6114 mm

Detector: Imaging plate

Pixel size: 200 $\mu$m

Measured angle: 0.001-0.1°

Exposure time: 300 sec

Standard sample: Collagen

Data processor: Rigaku R-AXIS

<SAXS (Short)>

Wavelength: 1.00 Å

Camera length: 1342 mm

Detector: Imaging plate

Pixel size: 200 $\mu$m

Measured angle: 0.01-2°

Exposure time: 300 sec

Standard sample: Behenic acid silver salt

Data processor: Rigaku R-AXIS

Diffraction patterns obtained from the above analysis are shown in FIGs. 5 and 6. Upon diffraction patterns obtained from the SAXS analysis, it is usually observed a scattering curve that precipitously rises up and moderately attenuates as the increase of diffraction angle (2θ). It is known that, when a long range periodic structure and a density fluctuation exist in a sample, a scattering curve does not uniformly and smoothly attenuate due to the scatterings induced thereby and, depending on samples, it may become to show a broken curve. As found in FIGs. 5 and 6, in any one of diffraction patterns, scattering curves smoothly attenuate without creating any kinks. These results show that, in the film of the particulate composition containing pullulan of the present invention that has an extremely low level of low-molecular weight components, i.e., it contains only less than 3% by weight of concomitant saccharides obtained by culturing MA446 strain, a long range periodic structure and a density fluctuation do not exist. Also, it reveals that the film is advantageous in terms of its intensity in that it does not have structural ununiformity such that the deformation of a specific part will increase when subjected to stress.

<Experiment 7: Content of component with maltotetraose structure in pullulan molecule>

A particulate composition containing pullulan, which had been obtained by culturing MA446 strain in the culture medium 3 according to the method in Experiment 2, and the particulate composition containing pullulan used as the control in Experiment 4 were respectively prepared into a 10 w/v % aqueous solution which was then admixed with 3-times volume of methanol, followed by promptly mixing the mixture and centrifuging the resulting mixture at 4°C and 15,700 g x 10 min. The resulting precipitate was redissolved in water and re-treated with the above procedure. The precipitate thus obtained was dried, dissolved in 20 mM acetate buffer (pH 5.0) to make into a 1 w/v % solution which was then admixed with "AMANO 3", a pullulanase specimen commercialized by Amano Enzyme Inc., Ltd., Aichi, Japan, in an amount of 5 units/g particulate composition, followed by an enzymatic reaction at 53°C for 48 hours. The resulting reaction solution was desalted, appropriately diluted, and analyzed on HPLC to determine the yields of maltotriose and maltotetraose. The HPLC analysis was conducted by using a column of MCI GEL CK04SS (8 mmφ inner diameter x 300 mm length, Mitsubishi Chemical Co., Ltd., Tokyo, Japan), refined water as an eluant, and a differential refractometer as a detector, and the elution conditions were 75°C and an eluant flow-rate of 0.6 ml/min. Based on the peak areas of maltotriose and maltotetraose obtained in the chromatogram, each content of the saccharides was determined and, based on the data, their molar ratio was determined. The results are in Table 8.

Table 8

| Particulate composition containing pullulan | Test powder | Control powder |
| --- | --- | --- |
| Maltotriose (G3)(%) | 97.8 | 97.2 |
| Maltotetraose (G4)(%) | 1.8 | 2.3 |
| G4/G3(molar %) | 1.4 | 1.8 |

As found in Table 8, the existing ratio (G4/G3) of the component with the maltotetraose structure in a pullulan molecule contained in the particulate composition containing pullulan, which had been obtained by culturing MA446 strain, was substantially the same as in the pullulan molecule in the particulate composition containing pullulan as a control (a control powder) . In the pullulan formed by S-2 strain and its mutant, the maltotetraose structure is present in a molar ratio of 1% to 3% against the maltotriose structure. As described above, the particulate composition containing pullulan obtained from the culture of MA446 strain makes a little difference in terms of the ratio of the maltotetraose structure against the maltotriose structure, compared to the particulate composition containing pullulan as the control powder. This reveals that the fact that, compared to conventional films of particulate compositions containing pullulan, the film of the particulate composition containing pullulan of the present invention has a higher rupture strength of piercing and a lesser incidence of cracking is not due to the structural difference but due to the concomitant saccharide content.
The process for producing the particulate composition containing pullulan of the present invention, the particulate composition obtained therewith, and the shaped products produced by using the particulate composition as a material or a part thereof are explained with reference to the following examples. The following never limit the scope of the present invention.

Example 1

<Particulate composition containing pullulan>

To 1,000 parts by weight of a culture medium (pH 7.0), which had been finally volumed up with water and contained as carbon sources 50 parts by weight of glucose, 50 parts by weight of maltose, and 50 parts by weight of a starch syrup ("MALT-RUP®", an enzymatically saccharified starch syrup, a solid content of 80%, a DE of about 47, commercialized by Hayashibara Shoji, Co., Ltd., Okayama, Japan) (a DE of about 67 as carbon sources), and two parts by weight of dipotassium hydrogen phosphate, two parts by weight of peptone, two parts by weight of sodium chloride, 0.4 part by weight of magnesium sulfate heptahydrate, and 0.01 part by weight of ferrous sulfate heptahydrate, was inoculated a seed culture of *Aureobasidium pullulans* MA446 strain, which had been cultured in a fresh preparation of the same culture medium as in the above at 27°C for 48 hours, followed by culturing the cells at 27°C for 72 hours under aeration and stirring conditions . The pullulan yield against the saccharides, which had been used as carbon sources, was 72.1% by weight. The cells were removed from the resulting culture by centrifugation, and the supernatant was decolored/ filtered with an activated charcoal, purified by desalting with an ion-exchange resin, concentrated, dried, and pulverized to obtain a particulate composition containing pullulan as a white powder having a satisfactory free-flowing ability.
The particulate composition had a pullulan content of 99.2% by weight, a concomitant saccharide content of 0.8% by weight, and a content of saccharides with glucose polymerization degrees of 3 to 90 of 0.3% by weight. Also, the particulate composition contains 0.2% by weight of mannitol, d.s.b. Further, the average molecular weight of the particulate composition was 428,000 daltons and the ratio of Mw/Mn was 35.3. The particulate composition can be formed into a film with a satisfactory rupture strength. In particular, the particulate composition has a lesser amount of concomitant saccharides, and the ratio of Mw/Mn as an index for broadness of molecular weight distribution is 35.3 as in the range of 10 to 70, indicating that it is expected to have a satisfactory stable properties in terms of physical properties such as the strength of shaped products, as well as its dissolution and disintegration rates. Because of these, the particulate composition can be used in foodproducts, as well as cosmetics, pharmaceuticals, quasi-drugs, etc. In particular, in such pharmaceuticals, the particulate composition can be preferably used as a pharmaceutical additive because it does not affect disposition of effective ingredients due to dispersion of solubility and disnintegrability.

Example 2

<Particulate composition containing pullulan>

Except for using, as carbon sources, 20 parts by weight of glucose, 20 parts by weight of maltose, and 100 parts by weight of a starch syrup (an acid saccharified starch syrup, solid content of 75%, DE of about 42, commercialized by Hayashibara Shoji, Co., Ltd., Okayama, Japan) (DE of about 54 as carbon sources), a particulate composition containing pullulan was obtained similarly as in Example 1. The pullulan yield against the saccharides, which had been used as carbon sources, was 70.4% by weight. The cells were removed from the resulting culture by centrifugation, and the supernatant was decolored/filtered with an activated charcoal, purified by desalting with an ion-exchange resin, concentrated, dried, and pulverized to obtain a particulate composition containing pullulan as a white powder having a satisfactory free-flowing ability.

The particulate composition had a pullulan content of 97.9% by weight, a concomitant saccharide content of 2.1% by weight, and a content of saccharides with glucose polymerization degrees of 3 to 90 of 0.7% by weight. Also, the particulate composition contains 0.6% by weight of mannitol, d.s.b. Further, the average molecular weight of the particulate composition was 401,000 daltons and the ratio of Mw/Mn was 41.1. The particulate composition can be formed into a film with a satisfactory rupture strength. In particular, the particulate composition has a lesser amount of concomitant saccharides, and the ratio of Mw/Mn as an index for broadness of molecular weight distribution is 41.1 as in the range of 10 to 70, indicating that it is expected to have a satisfactory stable properties in terms of physical properties such as the strength of shaped products, as well as its dissolution and disintegration rates. Because of these, the particulate composition can be used in food products, as well as cosmetics, pharmaceuticals, quasi-drugs, etc. In particular, in such pharmaceuticals, the particulate composition can be preferably used as a pharmaceutical additive because it does not affect disposition of effective ingredients due to dispersion of solubility and disnintegrability.

Example 3

<Pullulan film>

In 750 parts by weight of deionized water were dissolved 250 parts by weight of the particulate composition containing pullulan prepared in Example 1, and 0.5 part by weight of a surfactant (sucrose monolaurate) as a remover to obtain a material aqueous solution for pullulan film which was then deaerated *in vacuo.* The material aqueous solution was casted on over a plastic film and dried at 35°C and a relative humidity of 33% to obtain a pullulan film with a thickness of 50 $\mu$m. The pullulan film thus obtained had a moisture content of 3.5% by weight.

The pullulan film has an improved tolerance against a rupture stress and a high strength, has a satisfactory solubility in water, has a stable dissolution rate with a lesser dispersion independently of its production lot, and therefore it can be advantageously used in food products, cosmetics, pharmaceuticals, etc.

Example 4

<Mouth refreshing film>

According to conventional manner, to 69. 25 parts by weight of deionized water were added 22 parts by weight of a particulate composition containing pullulan obtained by the method in Example 1, one part by weight of carrageenan, 0.15 part by weight of xanthan gum, 0.15 part by weight of locust bean gum, 0.8 part by weight of maltitol, three parts by weight of "$\alpha$G-HESPERIDIN", a glycosyl hesperidin commercialized by Hayashibara Biochemical Laboratories, Inc., Okayama, Japan, 2.6 parts by weight of emulsified mint oil, 0.5 part by weight of a propolis extract, 0.3 part by weight of sucralose, and 0.25 part by weight of citric acid. The resulting mixture was dissolved by stirring at 90°C for three hours, casted on a 2 $\times$ 10 m stainless steel plate and dried at 60°C for four hours to obtain a film-shaped product with an about 200 $\mu$m thick, about 200 cm wide, 10 m length, about 8% moisture content, and about 2.2 g per 100 cm$^2$. The film-shaped product was cut into a size of 1 $\times$ 2 cm, and 20 sheets of which were packed in a portable container to obtain a mouth refreshing film.

Since the product is an edible film which has an appropriate strength and a prompt solubility in the mouth and contains glycosyl hesperidin and propolis extract, it is a cachou film that can be used for the purpose of maintaining/promoting the health in the mouth. The product is an edible film which is produced with the particulate composition containing pullulan of the present invention as a material that has a roughly constant saccharide composition being less varied batch-by-batch, and therefore the dissolution rate in the mouth is roughly constant, and the rate of dissolving the effective ingredients such as glycosyl hesperidin is usually stable.

Example 5

<Pullulan sheet>

Three hundred parts by weight of the particulate composition containing pullulan prepared in Example 2, 30 parts by weight of carboxy methyl cellulose, 10 parts by weight of L-ascorbic acid 2-glucoside, 0.1 part by weight of KANKOSO-401, three parts by weight of α-glucosyl rutin, five parts by weight of 1, 2-pentanediol, 2.5 parts by weight of N-coconut oil fatty acid acyl-L-sodium glutamate, one part by weight of potassium hydroxide, 0.3 part by weight of edetate trisodium, 0.3 part by weight of trisodium citrate, 0.2 part by weight of citric acid, and 1,000 parts by weight of ion-exchange water were made into a material aqueous solution for pullulan sheet which was then foamed. The material aqueous solution thus obtained was continuously casted on over a synthetic plastic film and dried by passing through a 50°C air stream to obtain a pullulan sheet with 100 μm thick.

Since the pullulan sheet is produced with the particulate composition containing pullulan of the present invention as a material that has a roughly constant saccharide composition being less varied batch-by-batch, and therefore it has an improved rupture strength, a satisfactory handleability, and an expected constant dissolution rate, independently of its production lot. Thus, the product can be suitably used as a processing material for cosmetic pack that allows effective ingredients such as L-ascorbic acid 2-glucoside and α-glucosyl rutin to act on the skin at a stable, constant rate.

Example 6

<Coated membrane>

An aqueous solution was prepared by dissolving in 100 parts by weight of water one part by weight of a particulate composition containing pullulan obtained by the method in Example 1 and 0.2 part by weight of gumarabic. A fresh egg within 10 hours of postlaying was soaked in the aqueous solution for 30 sec, taken out from the solution, dried at 30°C for two hours to form a pullulan membrane on the egg shell.

The egg coated with the pullulan membrane was stored at ambient temperature (15 to 25°C), and the edible period was compared with an untreated egg as a control (with no pullulan-membrane), revealing that the edible period of the egg coated with the pullulan membrane was prolonged even by about 5 to about 10 times. The pullulan membrane can be advantageously used to store eggs for use in materials for food industry, etc.

Example 7

<Sugar coated tablet>

A 150 mg crude agent as a wicking agent was coated with a coating solution which contained 50 parts by weight of crystalline maltitol, 20 parts by weight of a 10 w/w % aqueous solution prepared with a particulate composition containing pullulan produced by the method in Example 2, 15 parts by weight of water, 25 parts by weight of talc, and three parts by weight of titanium oxide until it reached about 230 mg weight. The resulting agent was further coated with another solution which contained 65 parts by weight of a fresh preparation of the same crystalline maltitol as used in the above, 10 parts by weight of a fresh preparation of the same 10 w/w % aqueous pullulan solution as used in the above, and 25 parts by weight of water, and successively coated with a liquid wax to obtain a glossy sugar coated tablet with a satisfactory appearance.

The product is a sugar coated tablet that is coated with a membrane containing pullulan, has an improved tolerance against rupture strength, has a lesser damage when transported or packed, and retains the quality of effective ingredients contained in the wicking agent for a relatively long period of time. Since the product is produced with the particulate composition containing pullulan of the present invention as a material that retains the saccharide composition in such a manner of being less varied batch-by-batch and roughly consistent, both the dissolution rate after administration and the binding with the wicking agent are roughly constant and the effective ingredients contained in the wicking agent are absorbed by the body at a stable, constant rate.

Example 8

<Tablet>

Five parts by weight of a particulate composition containing pullulan obtained by the method in Example 1, 30 parts by weight of a propolis extract, 20 parts by weight of "NYUKAOLIGO" LS-55P, a product name of a powder containing lactosucrose, commercialized by Hayashibara Shoji, Co., Ltd., Okayama, Japan, 10 parts by weight of calcium lactate, five parts by weight of L-ascorbic acid, one part by weight of α-glycosyl rutin, one part by weight of tribasic calcium phosphate, one part by weight of sucrose ester of fatty acid, and an adequate amount of a powdered flavor were mixed to homogeneity, and tabletted with a tablet press equipped with a punch having a diameter of 6 mm to obtain a tablet (about 300 mg/tablet).

The product is suitably used as an orally administrable product for promoting the health because it is a tablet that induces no crack when tabletted, has an adequate strength, exhibits a satisfactory dissolvability in water to ease its swallowing, and contains α-glycosyl rutin and propolis extract, as well as lactosucrose and pullulan. The adhesive force inherent to the particulate composition containing pullulan is roughly constant because the product is produced with the particulate composition containing pullulan of the present invention as a material that retains the saccharide composition in such a manner of being less varied batch-by-batch and roughly consistent. By tabletting prescribed ingredients under constant conditions, a satisfactory tablet, which has a usually-stable shape and strength, canbe obtained. The product is a tablet, which has a constant dissolution rate and disintegrability after administration and which the effective ingredients are absorbed by the body at a stable, constant rate.

Example 9

<Tablet>

According to conventional manner, 68 parts by weight of maltose, 15 parts by weight of α,α-trehalose, five parts by weight of a particulate composition containing pullulan obtained by the method in Example 2, five parts by weight of retinol palmitate, five parts by weight of ergocalciferol, 10 parts by weight of fursultiamine hydrochloride, five parts by weight of riboflavin, 10 parts by weight of pyridoxine hydrochloride, 10 parts by weight of tocopherol acetate, 30 parts by weight of nicotinic-acid amide, 0.01 part by weight of cyanocobalamin, 40 parts by weight of calcium pantothenate, 60 parts by weight of "AA2G", a product name of ascorbic acid 2-glucoside, commercialized by Hayashibara Biochemical Laboratories, Inc., Okayama, Japan, one part by weight of a flavor, and one part by weight of sodium monofluorophosphate were mixed to homogeneity and tabletted to obtain a tablet (200 mg/tablet).

Since the product does not induce any cracking when tabletted, has an adequate strength, and has a satisfactory water solubility, it is suitably used as a vitamin agent. The adhesive force inherent to the particulate composition containing pullulan is roughly constant because the product is produced with the particulate composition containing pullulan of the present invention as a material that retains the saccharide composition in such a manner of being less varied batch-by-batch and roughly consistent. By tabletting the prescribed ingredients under constant conditions, a satisfactory tablet, which has a usually-stable shape and strength, can be obtained. The product is a tablet, which has a constant dissolution rate and disintegrability after administration and which the effective ingredients are absorbed by the body at a stable, constant rate.

Example 10

<Tablet>

According to conventional manner, 450 parts by weight of ethenzamide, 300 parts by weight of acetaminophen, 50 parts by weight of caffeine, 25 parts by weight of maltitol, 25 parts by weight of α,α-trehalose, 200 parts by weight of sucrose, 400 parts by weight of xylitol, 500 parts by weight of corn starch, 20 parts by weight of polyethylene glycol, six parts by weight of a particulate composition containing pullulan prepared by the method in Example 1, and six parts by weight of gum arabic, one part by weight of "αG-SWEET", a product name of α-glucosyl stevioside commercialized by Toyo Sugar Refining Co., Ltd., Tokyo, Japan, were admixed and kneaded with 40 ml of water and tabletted with a tabletting machine to obtain a tablet (about 300 mg/tablet).

Since the product does not induce any cracking when tabletted, has an adequate strength, and has a satisfactory water solubility, it can be used as a sublingual-type cold medicine that is injectable while dissolving in the mouth. The adhesive force inherent to the particulate composition containing pullulan is roughly constant because the product is produced with the particulate composition containing pullulan of the present invention as a material that retains the saccharide composition in such a manner of being less varied batch-by-batch and roughly consistent. By tabletting the prescribed ingredients under constant conditions, a satisfactory tablet, which has a usually-stable shape and strength, can be obtained. Since the product has a constant dissolution rate in the mouth, the dissolution rates of the effective ingredients such as ethenzamide and acetaminophen are stable and they can be allowed to act on the body at a higher efficiency.

Example 11

<Granule>

One part by weight of "LS-90P", a product name of lactosucrose-containing-saccharides with a lactosucrose content of about 90% by weight, d.s.b., commercialized by Ensuiko Sugar Refining Co., Ltd., Tokyo, Japan, 0.1 part by weight of a particulate composition containing pullulan obtained by the method in Example 2, 0.5 part by weight of "αG-HESPERIDIN", a product name of glycosyl hesperidin, commercialzied by Hayashibara Biochemical Laboratories, Inc., Okayama, Japan, and 0.2 part by weight of "αG-SWEET", a product name of a glycosyl hesperidin specimen commercialized by Hayashibara Biochemical Laboratories, Inc., Okayama, Japan, were mixed to homogeneity and subjected to a granulator to obtain a granular powdered preparation.
Since the product contains pullulan, it has a satisfactory unity as a granular, improved safeness and dissolvability, and satisfactory swallowability. The product can be useful as a health food for maintaining/promoting the health because it contains glycosyl hesperidin. The adhesive force inherent to the particulate composition containing pullulan is roughly constant because the product is produced with the particulate composition containing pullulan of the present invention as a material that retains the saccharide composition in such a manner of being less varied batch-by-batch and roughly consistent. By subjecting to a granular machine the prescribed ingredients including the particulate composition under constant conditions, a satisfactory granule, which has a usually-stable shape and strength, can be obtained. The product is a granule, which has a constant dissolution rate and which the effective ingredients such as glycosyl hesperidin are stably absorbed by the body.

Example 12

<Fiber>

A particulate composition containing pullulan obtained in Example 1 was dissolved in water to obtain a 40 w/w % aqueous solution, into which were then dissolved 1.5 w/w %, d.s.b., of alginic acid and 0.02 w/w %, d.s.b., of locust bean gum, and the resulting solution was adjusted to 60°C, followed by pressing the solution into the atmospheric air through a cylindrical nozzle, 0.3 mm diameter, 1 mm length, at a pressure of 3 kg/cm$^2$ to form a strand and spooling it by a roller while drying to evaporate moisture.
The fiber thus obtained has a size of about 25 μm, and it can be twisted, knitted, and weaved. Also, the fiber has an adequate strength and hydrophilicity and it is characteristically, basically harmless and non stimulant to the skin because it is made from pullulan, it can be, for example, suitable for surgical strings, gauzes, etc. The adhesive force and dissolvability inherent to the particulate composition containing pullulan is roughly constant because the product is produced with the particulate composition containing pullulan of the present invention as a material that retains the saccharide composition in such a manner of being less varied batch-by-batch and roughly consistent. Accordingly, the product is an advantageous fiber that can form fibrous shaped products that have a constant quality such as strength and dissolvability under a prescribed spinning conditions.

Example 13

To one part by weight of a 0.01 w/w % solution of interferon-α with a specific activity of $1.5 \times 10^8$ units was added 100 parts by weight of a particulate composition containing pullulan obtained by the method in Example 1, and the resulting solution was aseptically membrane filtered, made free from pyrogens in usual manner, and distributed into 2 ml vials by 3,000,000 units per vial, followed by lyophilization.
The product has a relatively long shelf life even when stored at ambient temperature. The product promptly dissolves in water and it does not affect the disposition of interferon-α as the effective ingredient and retains it constantly because the product contains the particulate composition containing pullulan of the present invention wherein the saccharide composition less varies batch-by-batch and is kept roughly consistently. Thus, the product can be used as an injection, reagent for test, etc.

Industrial Applicability

As evident from the above explanation, according to the present invention, a particulate composition containing pullulan with an improved features can be produced from a culture obtained by culturing a mutant of *Aureobasidium pullulans* without employing a complicated purification step such as solvent precipitation. The particulate composition can be produced without a complicated purification step, has an extremely low level of concomitant saccharides in the total saccharides, has a relatively high purity of pullulan, has a stable composition, and has a molecular weight distribution of pullulan within a prescribed constant range. The shaped bodies such as films formed with the particulate composition containing pullulan of the present invention has a satisfactory, constantly stable strength, dissolvability, and disintegration independently of their production lots, thus the particulate composition will greatly contribute to the expansion of the use of pullulan in the fields of food products, as well as pharmaceuticals, quasi-drugs, and cosmetics, as a relatively low-price, high-quality particulate composition containing pullulan.

The present disclosure provides the following clauses:

Clause 1. A particulate composition comprising pullulan, characterized in that it is produced by a process comprising a step of culturing a mutant of a microorganism of the species *Aureobasidium pullulans* in a culture medium comprising glucose and maltose as carbon sources without employing a step of removing concomitant saccharides from the resulting culture, said particulate composition consisting of a pullulan fraction and a concomitant saccharide fraction, which are respectively insoluble and soluble in 75% by volume of methanol in water, wherein the ratio of the content of concomitant saccharides contained in said concomitant saccharide fraction against the content of the total saccharides in the whole of said particulate composition is 3% by weight or lower, when determined based on the anthrone sulfuric acid method; and said particulate composition containing mannitol.

Clause 2. The particulate composition of Clause 1, wherein the content of said mannitol does not exceed 2% by weight.

Clause 3. The particulate composition of Clause 1 or 2, which further has the following features (1) to (3):

(1) the weight-average molecular weight of said pullulan is about 50,000 to about 1,000,000 daltons;
(2) the ratio of the weight-average molecular weight against the number-average molecular weight of said pullulan is about 10 to about 70; and
(3) it contains a maltotetraose structure against a maltotriose structure in a molar ratio of 1% to 3% in the molecule of said pullulan.

Clause 4. The particulate composition of any one of Clauses 1 to 3, which further has the following feature (4):
(4) a film with a thickness of $500\mu$m, obtained by forming said particulate composition, does not exhibit any diffraction peak induced by a long range periodic structure and a density fluctuation when analyzed on small-angle X-ray scattering using a radiation light.

Clause 5. The particulate composition of any one of Clauses 1 to 4, which further has the following feature (5):
(5) a film with a thickness of 100 $\mu$m, obtained by forming said particulate composition, exhibits a rupture strength of piercing of 20 MPa or higher when analyzed on piercing test for rupture strength using an adaptor for piercing test with a sectional area of 1 mm$^2$.

Clause 6. The particulate composition of any one of Clauses 1 to 5, wherein said mutant of said microorganism of the species *Aureobasidium pullulans* is a mutant of *Aureobasidium pullulans* S-2 strain.

Clause 7. A process for producing said particulate composition containing pullulan of any one of Clauses 1 to 6, which comprises the steps of:

culturing a mutant of a microorganism of the species *Aureobasidium pullulans* in a culture medium comprising glucose and maltose as carbon sources;
removing the cells from the resulting culture; and decoloring, desalting, concentrating, and pulverizing the resultant;
wherein said process does not contain a step of removing concomitant saccharides.

Clause 8. The process of Clause 7, wherein said mutant of said microorganism of the species *Aureobasidium pullulans* is a mutant of *Aureobasidium pullulans* S-2 strain.

Clause 9. The process of Clauses 7 or 8, wherein said mutant of said microorganism of the species *Aureobasidium pullulans* is a mutant MA446 strain (deposited in International Patent Organism Depositary National Institute of Advanced Industrial Science and Technology under the accession number of FERM BP-11250).

Clause 10. A shaped product comprising pullulan, whenever prepared with said particulate composition of any one of Clauses 1 to 6 by using it at least as a partial material.

Clause 11. The shaped product of Clause 10, which is a food product, cosmetic, pharmaceutical, quasi-drug, or an intermediate thereof.

**Claims**

1.  A particulate composition comprising pullulan and concomitant saccharides containing mannitol, having the following features (1) to (5),

    (1) said pullulan and said concomitant saccharides are respectively insoluble and soluble in 75% by volume of methanol in water;
    (2) the content of said pullulan against the content of the total saccharides in the whole of said particulate composition is 97% by weight or higher, when determined based on the anthrone sulfuric acid method;
    (3) the content of said concomitant saccharides against the content of the total saccharides in the whole of said particulate composition is 3% by weight or lower, when determined based on the anthrone sulfuric acid method;
    (4) the content of said mannitol against the content of the total saccharides in the whole of said particulate composition does not exceed 2% by weight, on a dry solid basis; and
    (5) the weight-average molecular weight of said pullulan is 50,000 to 1,000,000 daltons.

2.  The particulate composition of claim 1, which further has the following feature (6):
    (6) the ratio of the weight-average molecular weight against the number-average molecular weight of said pullulan is 10 to 70.

3.  The particulate composition of claim 1, which further has the following feature (7):
    (7) the content of saccharides with a glucose polymerization degree of 3 to 90 contained in said concomitant saccharides against the content of total saccharides contained in the whole particulate composition is 2% by weight or lower, when determined based on the anthrone sulfuric acid method.

4.  The particulate composition of claim 1, which further has the following feature (8):
    (8) a film formed with said particulate composition to have a thickness of 100 $\mu$m exhibits a rupture strength of piercing of 20 MPa or higher when analyzed on piercing test for rupture strength using an adaptor for piercing test with a sectional area of 1 mm$^2$.

5.  A food, cosmetic, quasi-drug or pharmaceutical product comprising the particulate composition having features according to claim 1 to 4.

6.  A shaped product comprising the particulate composition having features according to claim 1 to 4.

FIG.1

FIG.2

FIG.3

FIG.4

FIG. 5

FIG. 6

30

Europäisches
Patentamt

European
Patent Office

Office européen
des brevets

# EUROPEAN SEARCH REPORT

**Application Number**

EP 21 19 2842

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 3 959 009 A (KATO KOSO ET AL) 25 May 1976 (1976-05-25) * column 4, lines 53-56; example * * column 3, lines 43-52 * ----- | 1-6 | INV. C08B37/00 C12P19/10 C12R1/645 A61K8/02 |
| Y | M R REKHA ET AL: "Pullulan as a Promising Biomaterial for Biomedical Applications: A Perspective", TRENDS BIOMATER. ARTIF. ORGANS, vol. 20, 2007, pages 1-6, XP055465164, * the whole document * ----- | 1-6 | A61K8/73 A61Q19/00 C08L5/00 C12N1/14 |
| Y | B.J. WILEY ET AL: "Control of Molecular Weight Distribution of the Biopolyrner Pullulan Produced by Aureobasidium pullulans", JOURNAL OF ENVIRONMENTAL POLYMER DEGRADATION, vol. 1, no. 1, 1993, pages 3-9, XP055465172, Netherlands * the whole document * ----- | 1-6 | |
| Y | SINGH RAM S ET AL: "Pullulan: Microbial sources, production and applications", CARBOHYDRATE POLYMERS, vol. 73, no. 4, 2008, pages 515-531, XP029237570, ISSN: 0144-8617, DOI: 10.1016/J.CARBPOL.2008.01.003 * the whole document * ----- | 1-6 | |

|  |  |
|---|---|
| | **TECHNICAL FIELDS SEARCHED (IPC)** |
| | C12P C08B A23L A61K C08L |

The present search report has been drawn up for all claims

| Place of search | Date of completion of the search | Examiner |
|---|---|---|
| Berlin | 15 November 2021 | Boeker, Ruth |

EPO FORM 1503 03.82 (P04C01)

## ANNEX TO THE EUROPEAN SEARCH REPORT
## ON EUROPEAN PATENT APPLICATION NO.

EP 21 19 2842

This annex lists the patent family members relating to the patent documents cited in the above-mentioned European search report.
The members are as contained in the European Patent Office EDP file on
The European Patent Office is in no way liable for these particulars which are merely given for the purpose of information.

15-11-2021

| Patent document cited in search report | | Publication date | Patent family member(s) | | Publication date |
|---|---|---|---|---|---|
| US 3959009 | A | 25-05-1976 | CA | 1029016 A | 04-04-1978 |
| | | | DE | 2507563 A1 | 28-08-1975 |
| | | | FR | 2262109 A1 | 19-09-1975 |
| | | | GB | 1459066 A | 22-12-1976 |
| | | | JP | S5414678 B2 | 08-06-1979 |
| | | | JP | S50116692 A | 12-09-1975 |
| | | | US | 3959009 A | 25-05-1976 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 59004291 A
- JP 59111817 A
- JP 5285969 A
- JP 57155975 A
- JP 2009539719 A
- JP 3350823 B
- JP 52006346 A
- JP 60016217 A
- JP 7000553 A
- JP 2005021124 A
- JP 51036360 A
- JP 51042199 A

- JP 55042635 A
- JP 58021739 A
- JP 55027099 A
- JP 58050665 A
- JP 60049164 A
- JP 3012917 B
- JP 3232488 A
- JP 4203322 B
- JP 2003096103 A
- JP 2004261132 A
- JP 3232488 B

**Non-patent literature cited in the description**

- **SATOSHI NAKAMURA.** Yu-Kagaku. *Oil Chemicals,* 1985, vol. 34 (10), 865-871
- **SATOSHI NAKAMURA.** *Japan Food Science,* 1986, vol. 25 (4), 61-67
- Food Industry. Korin Publishing Co., Ltd, 1987, vol. 30, 33-39
- Housou-Gijyutsu. *Packaging Technology,* 1991, vol. 29 (8), 852-859
- **YOSHINAO TANAKA.** *Convertech,* 2000, vol. 28 (9), 17-19
- Shoku-no-Kagaku. **KANOU TAKEUCHI.** Food Science. Korin Publisher, 2001, vol. 277, 96-99
- *Japan Food Science,* 1986, vol. 25 (4), 61-67

- Shokuhin-Kogyo. Food Industry. Korin Publishing Co., Ltd, 1987, vol. 30, 33-39
- Food Additive Pullulan. Hayashibara Shoji, Co., Ltd,
- Japanese Standard of Food Additives. Japan Food Additives Association (JAFA), 2007, 572-573
- Kogyo-Kagaku-Zasshi. *The Journal of Chemical Industry,* 1964, vol. 67, 757-760
- **SUMITA SUGIYAMA et al.** Shin-Pan-Biseibutsu-Kagaku-Jikken-Ho. Kodansha Scientific Ltd, 20 March 1999, 126-133
- Japanese Standard of Food Additives. Japan Food Additives Association, 2007, 572-573